# EUROPEAN PATENT SPECIFICATION

(11) **EP 0 538 401 B1**
(45) Date of publication and mention of the grant of the patent: **28.07.1999**
(21) Application number: 91914996.3
(22) Date of filing: 11.07.1991
(51) Int. Cl.: C12N 15/55, C12N 9/16, C12Q 1/68, G01N 33/577, C12N 1/21, C12N 5/10, C12Q 1/37

(54) **NOVEL RECEPTOR-TYPE PHOSPHOTYROSINE PHOSPHATASE**
PHOSPHOTYROSIN PHOSPHATASE ALS NEUER REZEPTOR-TYP
NOUVELLE PHOSPHOTYROSINE PHOSPHATASE DE TYPE RECEPTEUR

(30) Priority: 11.07.1990 US 551270; 26.02.1991 US 654188
(43) Date of publication of application: 28.04.1993
(73) Proprietor: NEW YORK UNIVERSITY, New York, NY 10016 (US)
(72) Inventor: SCHLESSINGER, Joseph, New York, NY 10011 (US)
(74) Representative: Thinat, Michel
(86) International application number: US9104892
(87) International publication number: WO9201050

(56) References cited:
- PROCEEDINGS OF THE NATL. ACADEMY OF SCIENCES USA, vol. 87, no. 12, June 1990, Washington, DC (US); R.J. MATTHEWS et al., pp. 4444-4448
- JOURNAL OF BIOLOGICAL CHEMISTRY, vol. 266, no. 19, 05 July 1991, Baltimore, MD (US); G. DAUM et al., pp. 12211-12215
- PROCEEDINGS OF THE NATL. ACADEMY OF SCIENCES USA, vol. 87, no. 16, August 1990, Washington, DC (US); J. SAP et al., pp. 6112-6116
- THE EMBO JOURNAL, vol. 9, no. 10, October 1990, Eynsham, Oxford (GB); N.X. KRUEGER et al., pp. 3241-3252
- PROCEEDINGS OF THE NATL. ACADEMY OF SCIENCES USA, vol. 87, no. 18, September 1990, Washington, DC (US); R. KAPLAN et al., pp. 7000-7004
- FEBS LETTERS, vol. 273, no. 12, 29 October 1990, Amsterdam (NL); F.R. JIRIK et al., pp. 239-242
- SCIENCE, vol. 230, 20 December 1985; R.K. SAIKI et al., pp. 1350-1354
- BIOCHEMISTRY, vol. 27, no. 24, 29 November 1988; N.K. TONKS et al., pp. 8695-8701
- JOURNAL OF EXPERIMENTAL MEDICINE, vol. 168, November 1988; M. STREULI et al., pp. 1523-1530
- PROCEEDINGS OF THE NATL. ACADEMY OF SCIENCES USA, vol. 86, November 1989, Washington, DC (US); M. STREULI et al., pp. 8698-8702
- JOURNAL OF BIOLOGICAL CHEMISTRY, vol. 264, no. 13, 05 May 1989; S.W. JONES et al., pp. 7747-7753
- EUROPEAN JOURNAL OF BIOCHEMISTRY, vol. 185, 06 November 1989; M.T. BUTLER et al., pp. 475-483
- R.K. SCOPES, "Protein Purification: Principles and Practice", 1987, Springer-Verlag, New York, NY (US); pp. 126-128
- PROCEEDINGS OF THE NATL. ACADEMY OF SCIENCES USA, vol. 76, no. 9, September 1979, Washington, DC (US); H. TOWBIN et al., pp. 4350-4354
- PROCEEDINGS OF THE NATL. ACADEMY OF SCIENCES USA, vol. 83, October 1986, Washington, DC (US); W.P. CARNEY et al., pp. 7485-7489

## Description

### BACKGROUND OF THE INVENTION

### Field of the Invention

The invention in the field of biochemistry and cell and molecular biology relates to novel receptor-type protein tyrosine phosphatase proteins or glycoproteins, termed R-PTPase-α, β and gamma, DNA coding therefor, methods for production and identification of the proteins, and methods for screening compounds capable of binding to and inhibiting or stimulating PTPase enzymatic activity.

### Description of the Background Art

The identification of several growth factor receptors and retroviral oncogenes as tyrosine-specific protein kinases indicated that protein phosphorylation on tyrosine residues plays a key role in cellular growth control. This notion has recently received support by the observation that the level of tyrosine phosphorylation of enzymes thought to play an important role in signal transduction (such as phospholipase C) correlates with their increased activity upon growth factor stimulation, thus establishing a functional role for tyrosine phosphorylation (Ullrich, A., et al., Cell 61:203-212 (1990)).

The degree and pattern of phosphorylation of tyrosine residues on cellular proteins are regulated by the opposing activities of protein-tyrosine kinases (PTKases; ATP:protein-tyrosine O-phosphotransferase, EC 2.7.1.112) and protein-tyrosine-phosphatases (PTPases; protein-tyrosine-phosphate phosphohydrolase, EC 3.1.3.48). The structural characteristics and evolution of PTKases as well as their role in the regulation of cell growth have been reviewed (Hunter, T., et al., Annu. Rev. Biochem. 54:897-930 (1985); Ullrich, A., et al., supra).

Tyrosine kinases comprise a discrete family of enzymes having common ancestry with, but major differences from, serine/threonine-specific protein kinases (Hanks, S.K. et al., (1988) Science 241, 42-52). The mechanisms leading to changes in activity of tyrosine kinases are best understood for receptor-type tyrosine kinases which have a transmembrane topology (Ullrich, A. et al., supra). With such kinases, the binding of specific ligands to the extracellular domain of these enzymes is thought to induce their oligomerization leading to an increase in tyrosine kinase activity and activation of the signal transduction pathways (Ullrich, A. et al., supra). The importance of this activity is supported by the knowledge that dysregulation of kinase activity through mutation or over-expression is a mechanism for oncogenic transformation (Hunter, T et al., supra; Ullrich, A. et al., 1990, supra).

The protein phosphatases are composed of at least two separate and distinct families (Hunter, T. Cell, 58:1013-1016 (1989)), the protein serine/threonine phosphatases and the protein tyrosine phosphatases. This is in contrast to protein kinases, which show clear sequence similarity between serine/threonine-specific and tyrosine-specific enzymes.

There appear to be two varieties of PTPase molecules. The first group is comprised of small, soluble enzymes that contain a single conserved phosphatase catalytic domain, and include (1) placental PTPase 1B (Charbonneau, H. et al., Proc. Natl. Acad. Sci. 86:5252-5256 (1989); Chernoff, J. et al., Proc. Natl. Acad. Sci. USA 87:2735-2789 (1990)), (2) T-cell PTPase (Cool, D.E. et al., Proc. Natl. Acad. Sci. USA 86:5257- 5261 (1989)), and (3) rat brain PTPase (Guan, K., et al., Proc. Natl. Acad. Sci. USA, 87:1501- 1505 (1990).

The second group is made up of the more complex, receptor-linked PTPases, termed R-PTPases, which are of high molecular weight and contain two tandemly repeated conserved domains separated by 56-57 amino acids. One example of R-PTPases are the leukocyte common antigens (LCA) (Ralph, S.J., EMBO J., 6:1251-1257 (1987); Charbonneau, H., et al., Proc. Natl. Acad. Sci. USA, 85:7182-7186 (1988)). LCA, also known as CD45, T200 and Ly-5 (reviewed in Thomas, M.L., Ann. Rev. Immunol. 7:339-369 (1989)) comprises a group of membrane glycoproteins expressed exclusively in hemopoietic (except late erythroid) cells, derived from a common gene by alternative splicing events involving the amino terminus of the proteins. Whereas the precise function of CD45 is unknown, many studies have implicated these antigens in a number of processes, including the activity of cytotoxic T lymphocytes and natural killer cells, IL-2 receptor expression, B-cell differentiation, and T lymphocyte proliferation (Pingel, J.T. et al., Cell 58:1055-1065 (1989)).

Other examples of R-PTPases are the LCA-related protein, LAR (Streuli, M., et al., J. Exp. Med., 168:1523-1530 (1988)), and the LAR-related Drosophila proteins DLAR and DPTP (Streuli, M., et al., Proc. Natl. Acad. Sci. USA, 86:8698-8702 (1989)). Jirik et al. screened a cDNA library derived from the human hepatoblastoma cell line, HepG2, with a probe encoding the two PTPase domains of LCA (FASEB J. 4:A2082 (1990), abstr. 2253) and discovered a cDNA clone encoding a new R-PTPase, named He-PTP. The HePTP gene appeared to be expressed in a variety of human and murine cell lines and tissues.

While we are beginning to understand more about the structure and diversity of the PTPases, much remains to be learned about their cellular functions. It has been suggested (Tonks, N.K., et al., Biochemistry27:8695-8701 (1988)) that the small, soluble PTPase enzymes may have a "housekeeping" function. On the other hand, the R-PTPases would be expected to be more restricted in their activities because of their location in the cell membrane and their potential regulation by extracellular ligands. Regarding the role of LCA (CD45) in T cells, it was found that T cell clones deficient in the expression of LCA failed to proliferate when stimulated by a specific antigen or by cross-linking of CD3 (Pingel, J.T., et al., supra). PTPase cross-linking inhibits T cell receptor CD3-mediated activation in human T cells (Kiener, P.A. et al., J. Immunol. 143:23-28 (1989)). The PTPase activity of LCA plays a role in the activation of pp56^{lck}, a lymphocytespecific PTKase (Mustelin, T., et al., Proc. Natl. Acad. Sci. USA, 86:6302-6306 (1989); Ostergaard, H.L., et al., Proc. Natl. Acad. Sci. USA, 86:8959-8963 (1989)). These authors hypothesized that the phosphatase activity of LCA activates pp56^{lck} by dephosphorylation of a C-terminal tyrosine residue, which may, in turn, be related to T-cell activation.

Using site-directed mutagenesis to determine which of four conserved cysteines in LCA (two per phosphatase domain) was required for enzyme activity toward artificial substrates, Streuli et al. (1989, supra) found that only one cysteine residue (residue 177 of LCA phosphatase domain-1) of LCA was essential for activity, indicating that, most likely, only the first phosphatase domain has enzymatic activity. However, the possibility that the second domain can dephosphorylate a different substrate was not excluded. More recently, Streuli et. al. (EMBO J., 9:2399-2407 (1990)) determined that the second conserved domain of LCA (and of LAR) lacked detectable phosphatase activity but sequences within the domain could influence substrate specificity.

In order to better understand and to be able to control phosphotyrosine metabolism, one must comprehend not only the role of kinase activity, but also the action of phosphatase enzymes as well. Elevation of cellular phosphotyrosine may occur through mechanisms not involving the activation of a tyrosine kinase itself. For instance, expression of the v-crk oncogene, though not a tyrosine kinase itself, induces the phosphorylation of tyrosine residues through a poorly understood mechanism (Mayer, B.J. et al. (1988) Nature 332, 272-275). Potentially, such an outcome could result from either mutation of the substrate or through a general decrease in cellular phosphatase activity, especially in view of the normally high turnover rate of cellular tyrosine-phosphate (Sefton, B.M. et al. (1980) Cell 20, 807-816). The latter possibility is suggested by the demonstration that tyrosine phosphatase inhibitors can "reversibly transform" cells (Klarlund, J.K. Cell 41: 707-717 (1985)). PTPases could therefore be viewed as potential recessive oncogenes.

It is becoming clear that dephosphorylation of tyrosine can by itself function as an important regulatory mechanism. Dephosphorylation of a C-terminal tyrosine residue stimulates tyrosine kinase activity in the src-family of tyrosine kinases (Hunter, T. (1987) Cell 49, 1-4). Tyrosine dephosphorylation has been suggested to be an obligatory step in the mitotic activation of the MPF (maturation promoting factor) kinase (Morla, A.O. et al. (1989) Cell 58, 193-203). Lastly, mutant analysis of primitive eukaryotes has established crucial roles for serine phosphatase in cellular physiology (Cyert, M.S. et al. (1989) Cell 57, 891-893). These observations point out the need in the art for increasing our understanding of the mechanisms that regulate tyrosine phosphatase activity.

It is clear in the art that further analysis of structure-function relationships among these membrane receptors are needed to gain important understanding of the mechanisms of cell growth, differentiation, and oncogenesis.

### SUMMARY OF THE INVENTION

The inventors have conceived of a role for R-PTPases in cellular control mechanisms, both as potential anti-oncogenes, and as effectors in a newly discovered mechanism of trans-membrane signalling. They therefore undertook a search for an R-PTPase potentially involved in such processes, and describe herein the identification of a novel, widely expressed member of the R-PTPase family, which has a trans-membrane topology. The novel R-PTPase, in a manner analogous to receptor tyrosine kinases, is subject to direct regulation by a variety of different extracellular ligands.

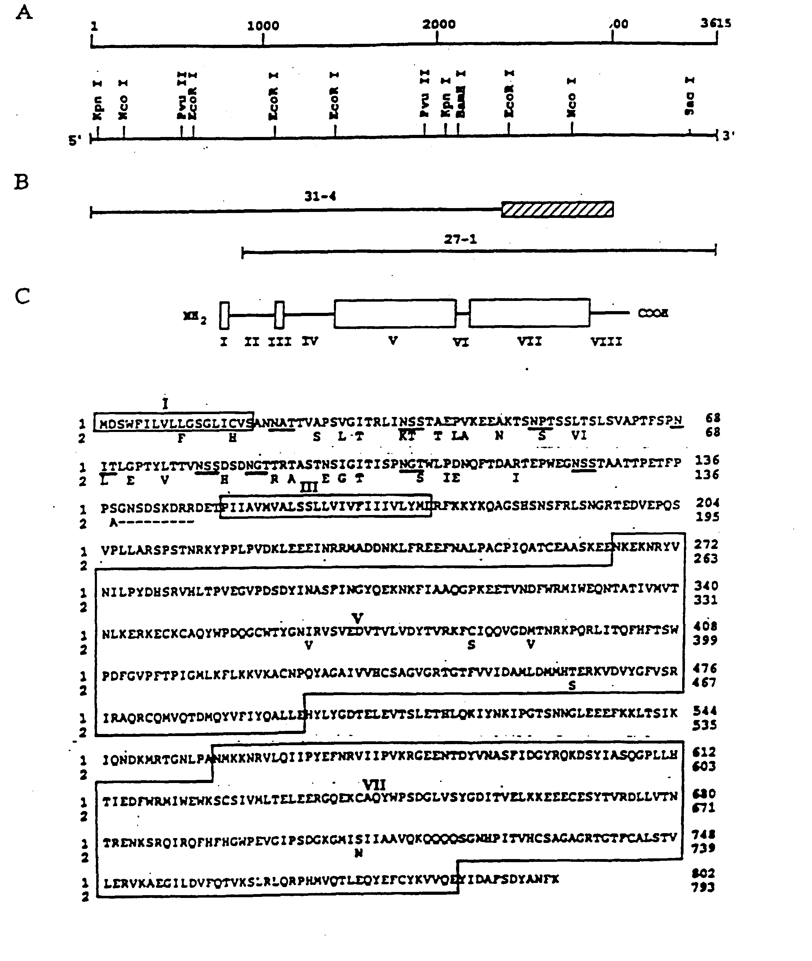

The present invention thus provides a human receptor-type protein tyrosine phosphatase-α (R-PTPase-α) protein as described in line 1 of Figure 4 other than leucocyte, common antigen (LCA or CD45) and leucocyte common antigen-related protein (LAR), a functional derivative of the human R-PTPase-α or a homolog of the human R-PTPase-α in another mammalian species. When the molecule is of natural origin, it is substantially free of other proteins or glycoproteins with which it is natively associated. This naturally-occurring molecule is normally present in mammalian liver, kidney and brain. Alternatively, the R-PTPase-α molecule may not be of natural origin, such as one prepared by chemical or recombinant means.

The substantially pure R-PTPase-α protein or glycoprotein of the invention may be produced by biochemical purification of the glycoprotein of natural origin ; alternatively, the R-PTPase-α may be produced by recombinant means in prokaryotic or eukaryotic hosts.

In particular, the invention is directed to the molecule R-PTPase-α having the amino acid sequence of Figure 4, or a functional derivative thereof.

A DNA molecule consisting essentially of a nucleotide sequence encoding R-PTPase-α of mouse or human origin, or R-PTPase-β or R-PTPase-gamma, both of human origin, or a functional derivative thereof, in the form of cDNA or genomic DNA are also disclosed here. The invention is further directed to the DNA sequence in the form of an expression vehicle, as well as prokaryotic and eukaryotic hosts transformed with the DNA.

Also included in the present invention is a process for preparing an R-PTPase-α protein or glycoprotein of this invention, or a functional derivative thereof, comprising:
(a) culturing a host capable of expressing the protein under culturing conditions,
(b) expressing the protein ; and
(c) recovering the protein from the culture.

The invention is also directed to an antibody, polyclonal, monoclonal, or chimeric, specific for the R-PTPase-α protein or glycoprotein.

The invention is also directed to a method for detecting the presence of nucleic acid encoding a normal or mutant R-PTPase-α in a subject comprising :
(a) contacting a cell or an extract thereof from the subject with an oligonucleotide probe encoding at least a portion of the normal or mutant R-PTPase-α under hybridizing conditions; and
(b) measuring the hybridizing of the probe to the nucleic acid of the cell, thereby detecting the presence of the nucleic acid.

The DNA can be selectively amplified, using the polymerase chain reaction, prior to assay.

The invention is further directed to a method for detecting the presence, or measuring the quantity of an R-PTPase-α in cell or in a subject comprising :
(a) contacting said cell or an extract thereof with an antibody specific for an epitope of the R-PTPase ; and
(b) detecting the binding of the antibody to the cell or extract thereof, or measuring the quantity of antibody bound,
thereby detecting the presence or measuring the quantity of the R-PTPase-α.

The present invention is also directed to methods for identifying and isolating a compound capable of binding to an R-PTPase-α from a chemical or biological preparation comprising :
(a) attaching the R-PTPase-α or the ligand-binding portion thereof to a solid phase matrix ;
(b) contacting the chemical or biological preparation with the solid phase matrix allowing the compound to bind, and washing away any unbound material ;
(c) detecting the presence of the compound bound to the solid phase ; and, for purposes of isolation,
(d) eluting the bound compound, thereby isolating the compound.

Finally, the invention includes a method for identifying a compound capable of stimulating or inhibiting the enzymatic activity of a R-PTPase-α, comprising :
(a) contacting the compound with the R-PTPase-α in pure form, in a membrane preparation, or in a whole live or fixed cell ;
(b) incubating the mixture in step (a) for a sufficient interval ;
(c) measuring the enzymatic activity of the R-PTPase-α ;
(d) comparing the enzymatic activity to that of the R-PTPase-α incubated without the compound,
thereby determining whether the compound stimulates or inhibits the activity.

### BRIEF DESCRIPTION OF THE DRAWINGS

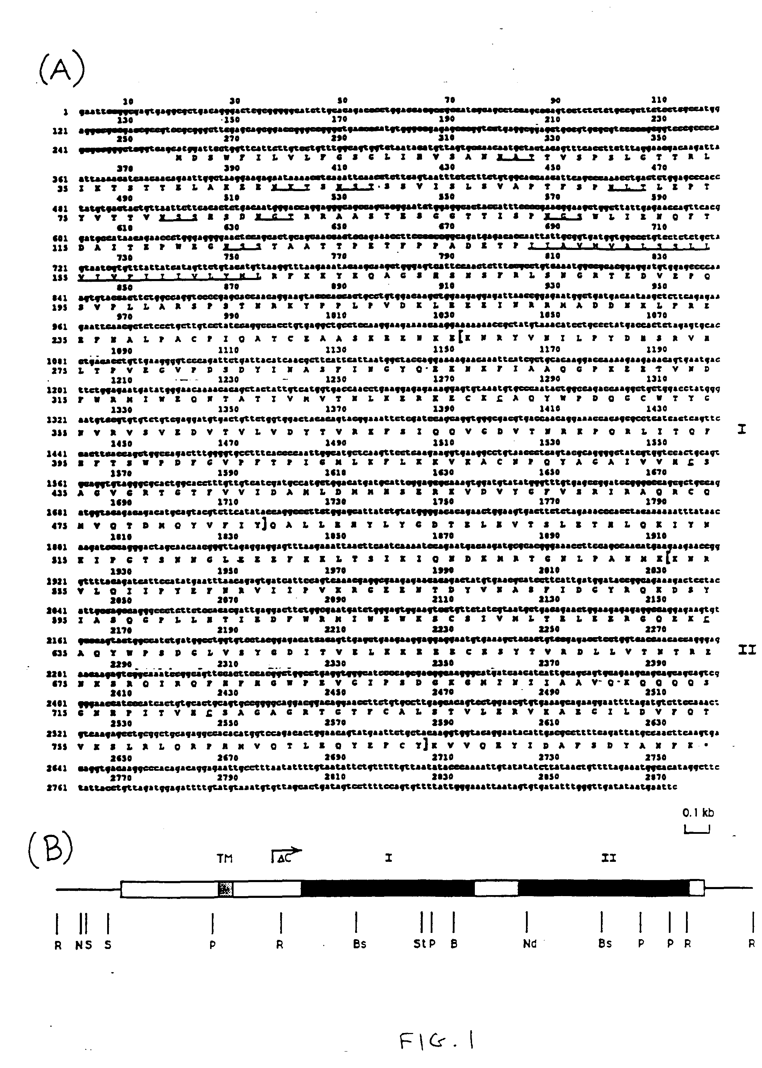

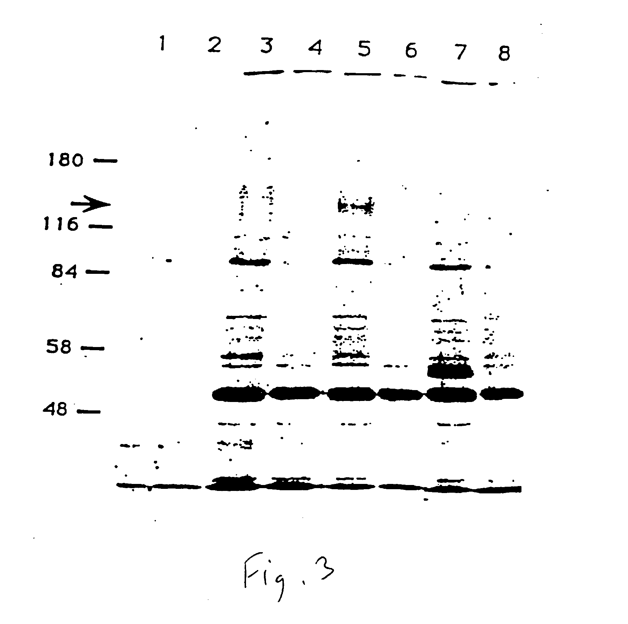

Figure 1 presents the predicted primary structure of murine R-PTPase-α. Panel (a) shows the frequence of the phage lambda-109 cDNA insert and predicted R-PTPase-α protein sequence (the standard one-letter amino acid code is used). The initiation codon ATG is shown in italics, and the stop codon is indicated by an asterisk. The putative trans-membrane domain (amino acids 143 to 166) is underlined as well as the potential N-linked glycosylation sites in the extracellular domain. The borders of homology between the tandemly repeated PTPase domains (I and II) are indicated by square brackets. Cysteine residues conserved in the catalytic domain of all known R-PTPases are also underlined. Panel (b) shows a schematic structure of a lambda-109 cDNA clone containing the R-PTPase-α coding sequence. R-PTPase domains I and II are indicated as black boxes, the trans-membrane domain is shaded. The start of the N-terminally truncated PTP-DeltaC protein menticned in Figure 3, below is indicated by an arrow (at amino acid 214). The positions of restriction sites used for generating nested deletions for sequencing are indicated. Abbreviations : TM, trans-membrane domain ; B, BamHI site ; Bs, BstEII site ; N, NcoI site ; Nd, NdeI site ; P, PstI site ; R, EcoRI site ; S : SacII site ; St, StuI site.

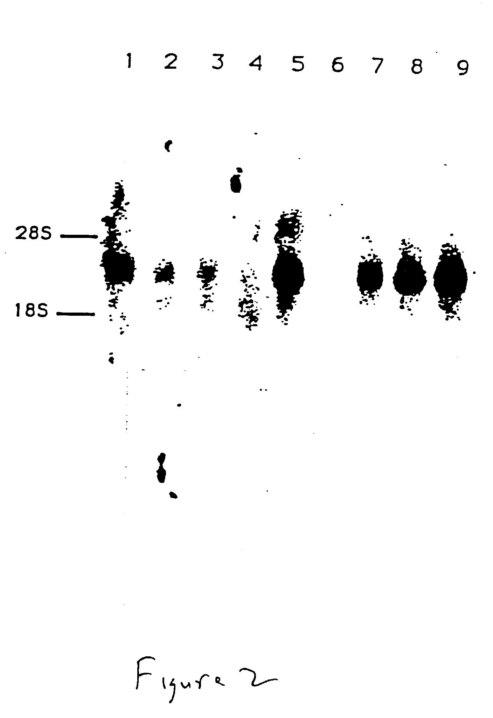

Figure 2 is a diagram of a Northern blot showing expression of the murine R-PTPase-α mRNA. 5 µg of Poly A⁺ RNA from mouse tissues and cell lines was fractionated on formaldehyde-containing agarose gels and subjected to Northern analysis using as a probe the entire R-PTPase-α cDNA. The positions of the 28S and 18S ribosomal RNA is indicated.
Lanes: 1, kidney; 2, lung; 3, heart; 4, stomach; 5, brain; 6, spleen; 7, liver; 8, NIH-3T3 fibroblast cell line (Honegger, A.M. et al. (1987) Cell 51, 199-209); 9, BAF prepro-B lymphoid cell line (Palacios, R. et al. (1985) Cell 41, 727-734).

Figure 3 is a diagram showing results of PAGE of immunoprecipitates of the murine R-PTPase-α protein. COS cells were transiently transfected using the DEAE-dextran method with a negative control plasmid (expression vector pLSV without insert), with either pLSV-PTP-α (the same expression vector containing the R-PTPase-α cDNA), or with the expression vector pLSVDeltaC, designed to express a truncated R-PTPase-α protein (PTP-DeltaC, amino-acids 214-794) from which the transmembrane and extracellular domains have been removed (an initiator methionine residue was introduced at this position using site-directed mutagenesis). After metabolic labelling with [³⁵S]-methionine, immunoprecipitation was performed using either pre-immune serum (lanes 1 and 2) or with an antiserum (2A) (lanes 3-8), raised against a synthetic peptide corresponding to the C-terminus of the R-PTPase-α protein in the absence or presence of 100 µg of the immunizing peptide. Sizes of molecular weight markers are shown in kDa. The arrow marks the position of the 130 kD R-PTPase-α protein (lane 5).
Lanes are: 1: pLSV, pre-immune serum; 2: pLSV-PTP-α, pre-immune serum; 3: pLSV, antiserum 2A; 4: pLSV, antiserum 2A in the presence of synthetic peptide; 5: pLSV-PTP-α, antiserum 2A; 6: pLSV-PTP-α, antiserum 2A in the presence of synthetic peptide; 7: pLSVDeltaC, antiserum 2A; 8: pLSVDeltaC, antiserum 2A in the presence of synthetic peptide.

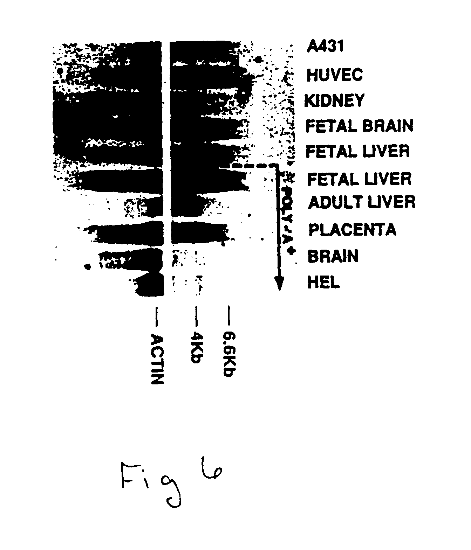

Figure 4 shows the structure of human R-PTPase-α deduced from the sequence of cDNA clones.
(A) Composite restriction map [3615 base pairs (bp)] of overlapping clones 31-4 and 27-1, which together contain the entire coding region of human R-PTPase-α.
(B) Relative positions of clones 31-4 and 27-1. Both strands of each clone were sequenced in their entirety by using a series of oligonucleotide primers. The hatched region in clone 31-4 corresponds to the fragment used as probe for the Northern blot (Figure 6, below) as well as for the chromosome assignment.
(C) Comparison of the amino acid sequences of human (line 1) and mouse (line 2) R-PTPase-α. The single-letter amino acid code is used. Only the differences are shown. The dashed line indicates a stretch of amino acids not present in the mouse sequence. The coding portion of human R-PTPase-α, and its position relative to clones 31-4 and 27-1 (B), is shown at the top. The following regions are designated: signal peptide (I), extracellular domain with potential N-glycosylation sites for the human protein underlined (II), transmembrane (III), juxtamembrane (IV), first phosphatase domain (V), interdomain (VI), second phosphatase domain (VII) , C terminus (VIII).

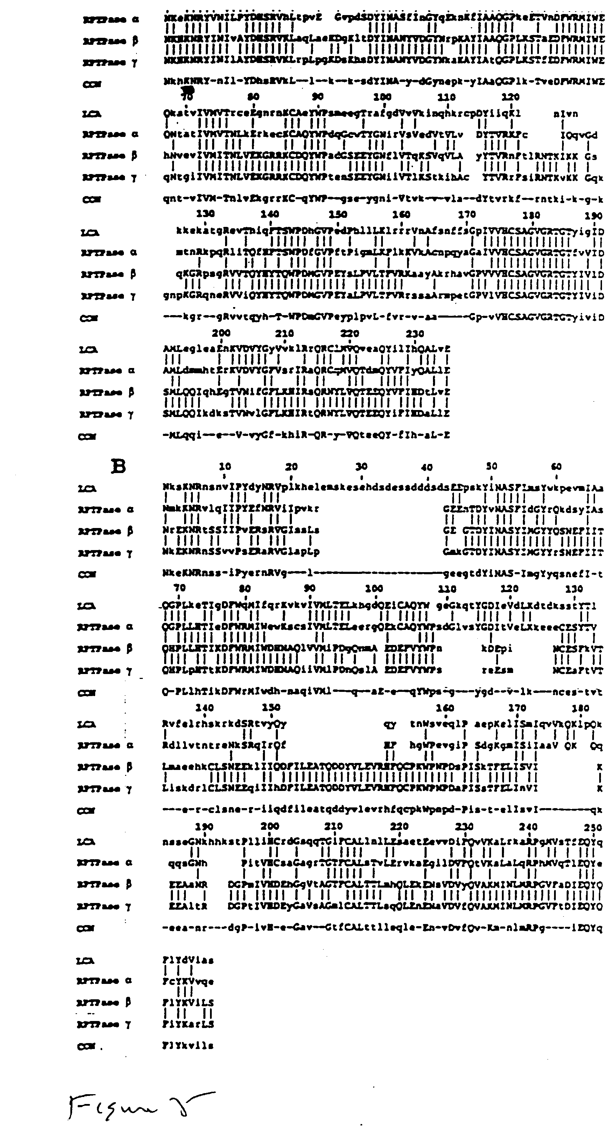

Figure 5 shows a comparison of the amino acid sequences of the first (A) and second (B) conserved phosphatase of human R-PTPases LCA, a, β and gamma. CON is the consensus sequence: a capital letter indicates complete agreement, while a small letter indicates agreement among two or three of the four sequences. A dash indicates lack of consensus.

Figure 6 shows a gel pattern indicating relative expression of human R-PTPase-α in various tissues and cell lines, as determined by Northern blot hybridization with R-PTPase-α probe (Upper) and β-actin probe (Lower). Total RNA (five left lanes) or poly (A)⁺ RNA (five right lanes) samples from the indicated human cell lines or tissues were analyzed. A431 is a human epidermoid carcinoma cell line; HEL is an erythroleukemia cell line; all other lanes represent flash-frozen tissues samples (HUVEC - human umbilical vein endothelial cells).

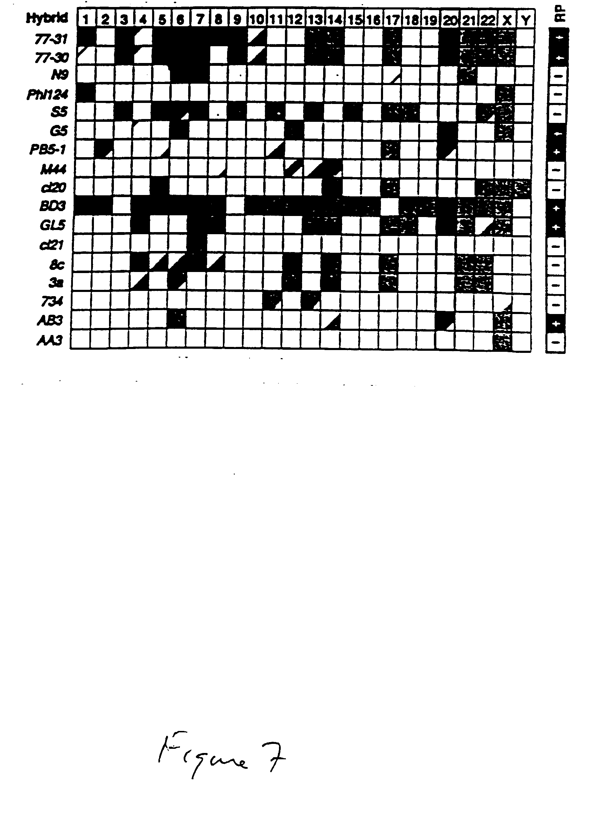

Figure 7 is a matrix diagram which shows the chromosomal localization of human R-PTPase-α based on analysis of a panel of 17 rodent-human somatic cell hybrids. A completely stippled box indicates that the hybrid contained the chromosome indicated in the upper row; lower-right stippling indicates presence of the long arm (or part of the long arm, indicated by a smaller fraction of stippling) of the chromosome; upper-left stippling indicates presence of the short arm (or partial short arm) of the chromosome; an open box indicates absence of the chromosome. The column for chromosome 20 is boldly outlined and stippled to highlight correlation of presence of this chromosome (or chromosome region) with the presence of the R-PTPase-α gene. The pattern of retention of the human R-PTPase-α sequences in the hybrids is shown at right (RPTPα): presence of the gene is indicated by a "+" in a stippled box; absence of the gene is indicated by a "-" in an open box.

### DESCRIPTION OF THE PREFERRED EMBODIMENTS

Through the use of recombinant DNA methods, the present inventors have identified novel mammalian receptor-type (transmembrane) protein tyrosine phosphatases (PTPase; EC 3.1.3.48). The murine R-PTPase-α has 794 amino acids, whereas the human R-PTPase-α has 802 amino acids. In view of its receptor-like structure, and the likelihood that it is part of a family, the inventors have termed this protein, R-PTPase-α (receptor protein tyrosine phosphatase alpha). The family is designated herein as the "R-PTPases."

R-PTPase-α has an intracellular domain homologous to the catalytic domains of other tyrosine phosphatases. The inventors have further characterized the 142 amino acid extracellular domain (including signal peptide) as having a high serine and threonine content (32%) and 8 potential N-glycosylation sites. The inventors have produced cDNA clones coding for the novel protein, and expressed the protein from eukaryotic hosts. Northern analysis has been used to identify the natural expression of the protein in various cells and tissues. They have further produced a polyclonal antibody to the protein by immunization with a synthetic peptide of R-PTPase-α, which identifies a 130 kDa protein in cells transfected with a cDNA clone encoding a portion of R-PTPase-α.

Remarkably, in addition to being composed of intracellular domains having enzymatic activity, the receptor family to which R-PTPases belong includes transmembrane proteins having N-terminal extracellular domains; this is analogous to the tyrosine kinase enzyme family (Tonks, N.K. et al. (1988) Biochemistry 27, 8695-8701; Charbonneau, H. et al. (1988) Proc. Natl. Acad. Sci. USA 85, 7182-7186; Streuli, M. et al., (1988) J. Exp. Med. 168, 1523- 2530; Streuli, M. et al., (1989) Proc. Natl. Acad. Sci. USA 86, 8698-8702). The present inventors have therefore concluded that ligands in the extracellular environment can control the activity of this membrane-associated subclass of PTPases.

R-PTPase-α and the other R-PTPases of the present invention are useful in methods for screening drugs and other agents which are capable of activating or inhibiting the R-PTPase activity, and thereby affecting major pathways of cellular metabolism. By attaching an intact R-PTPase, or the ligand-binding portion thereof, to a solid phase matrix, an affinity probe is created which can be used to screen biological products or chemical agents for their capacity to interact with the receptor on the basis of their binding activity. Bound material can then be eluted from the affinity probe in purified form.

Methods for coupling proteins and peptides to the solid phase, the solid phase substances useful in these methods, and means for elution, are well known to those of skill in the art.

The R-PTPase protein or derivatives thereof having enzymatic activity can be used for testing of compounds capable of enhancing or inhibiting the phosphatase activity. The ability of a compound to modify phosphatase activity can be tested in an in vitro system wherein the test compound is added to purified R-PTPase protein or enzymatically active derivatives thereof, and the effects on enzyme activity measured using standard enzymological procedures well known to those of skill in the art.

Alternatively, the action of a compound on R-PTPase activity can be measured in a whole cell preparation using live or fixed cells, or a membrane fraction derived from live or fixed cells. This method is useful for screening compounds acting via the extracellular receptor portion of the protein, as well as compounds acting directly on the enzymatic portion of the protein. A test compound is incubated with cells, or with a membrane preparation derived therefrom, which express high amounts of the R-PTPase of this invention, such as transfected COS or NIH-3T3 cells. The amount of cellular phosphotyrosine is then measured, using methods well-known in the art (Honegger, A.M. et al., Cell 51:199-209 (1987); Margolis, B. et al., Cell 57:1101-l107 (1989)). The results are compared to results obtained in the absence of the test compound, or in the absence or presence of a known activator of R-PTPase. In such studies, the action of the test compound in the presence of an activator of tyrosine kinase can also be measured.

A compound which stimulates R-PTPase activity will result in a net decrease in the amount of phosphotyrosine, whereas a compound which inhibits R-PTPase activity will result in a net increase in the amount of phosphotyrosine.

In the case of growth factor receptors which are tyrosine kinases, such as the receptors for epidermal growth factor (EGF) and for platelet-derived growth factor (PDGF), tyrosine phosphorylation is linked to cell growth and to oncogenic transformation. Activation of a PTPases, leading to dephosphorylation, would serve as a counterregulatory mechanism to prevent or inhibit growth, and might serve as an endogenous regulatory mechanism against cancer. Thus, mutation or dysregulation of this receptor/enzyme system may promote susceptibility to cancer

The insulin receptor is also a tyrosine kinase, and phosphorylation of tyrosine in cells bearing insulin receptors would be associated with normal physiological function. In contrast to the case of cell growth and cancer, activation of an R-PTPase would counteract insulin effects. Subnormal R-PTPase levels or enzymatic activity would act to remove normal counterregulatory mechanisms. Perhaps more important, though, over-activity, or inappropriate activation, of a R-PTPase would be expected to inhibit or totally prevent the action of insulin on cells, leading to diabetes (of an insulin-resistant variety). Thus, susceptibility to diabetes may be associated with R-PTPase dysregulation.

Therefore, the methods of the present invention for identifying normal or mutant R-PTPase genes, or for measuring the amount or activity of R-PTPase associated with a cell or tissue, can serve as methods for identifying susceptibility to cancer, diabetes, or other diseases associated with alterations in cellular phosphotyrosine metabolism.

The present invention provides methods for evaluating the presence and the level of normal or mutant R-PTPase in a subject. Absence, or more typically, low expression of the R-PTPase, or presence of a mutant R-PTPase, in an individual may serve as an important predictor of susceptibility to oncogenic transformation and the development of cancer. Alternatively, over-expression of R-PTPase, possibly due to a mutant receptor/enzyme system insensitive to negative regulation, or due to overabundance of a stimulatory ligand in the body, may serve as an important predictor of susceptibility to diabetes.

Oligonucleotide probes encoding various portions of the R-PTPase (see below) are used to test cells from a subject for the presence of DNA or RNA sequences encoding the R-PTPase. A preferred probe would be one directed to the nucleic acid sequence encoding at least 4 amino acid residues, and preferably at least 5 amino acid residues, of the R-PTPase-α or other R-PTPase protein of the present invention. Qualitative or quantitative assays can be performed using such probes. For example, Northern analysis (see Examples III and VI, below) is used to measure expression of an R-PTPase mRNA in a cell or tissue preparation.

Such methods can be used even with very small amounts of DNA obtained from an individual, following use of selective amplification techniques. Recombinant DNA methodologies capable of amplifying purified nucleic acid fragments have long been recognized. Typically, such methodologies involve the introduction of the nucleic acid fragment into a DNA or RNA vector, the clonal amplification of the vector, and the recovery of the amplified nucleic acid fragment. Examples of such methodologies are provided by Cohen et al. (U.S. Patent 4,237,224), Sambrook et al. Molecular Cloning: A Laboratory Manual, Second Edition, Cold Spring Harbor Press, Cold Spring Harbor, NY (1989), which references are herein incorporated by reference).

Recently, an in vitro, enzymatic method has been described which is capable of increasing the concentration of such desired nucleic acid molecules. This method has been referred to as the "polymerase chain reaction or "PCR" (Mullis, K. et al., Cold Spring Harbor Symp. Ouant. Biol. 51:263-273 (1986); Erlich, H. et al., EP 50,424; EP 84,796, EP 258,017, EP 237,362; Mullis, K., EP 201,184; Mullis, K. et al., US 4,683,202; Erlich, H., US 4,582,788; and Saiki, R. et al., US 4,683,194).

The polymerase chain reaction provides a method for selectively increasing the concentration of a particular nucleic acid sequence even when that sequence has not been previously purified and is present only in a single copy in a particular sample. The method can be used to amplify either single- or double-stranded DNA. The essence of the method involves the use of two oligonucleotide probes to serve as primers for the template-dependent, polymerase mediated replication of a desired nucleic acid molecule.

The precise nature of the two oligonucleotide probes of the PCR method is critical to the success of the method. As is well known, a molecule of DNA or RNA possesses directionality, which is conferred through the 5'-3' linkage of the phosphate groups of the molecule. Sequences of DNA or RNA are linked together through the formation of a phosphodiester bond between the terminal 5' phosphate group of one sequence and the terminal 3' hydroxyl group of a second sequence. Polymerase dependent amplification of a nucleic acid molecule proceeds by the addition of a 5' nucleotide triphosphate to the 3' hydroxyl end of a nucleic acid molecule. Thus, the action of a polymerase extends the 3' end of a nucleic acid molecule. These inherent properties are exploited in the selection of the oligonucleotide probes of the PCR. The oligonucleotide sequences of the probes of the PCR method are selected such that they contain sequences identical to, or complementary to, sequences which flank the particular nucleic acid sequence whose amplification is desired.

More specifically, the oligonucleotide sequences of the "first" probe is selected such that it is capable of hybridizing to an oligonucleotide sequence located 3' to the desired sequence, whereas the oligonucleotide sequence of the "second" probe is selected such that it contains an oligonucleotide sequence identical to one present 5' to the desired region. Both probes possess 3' hydroxy groups, and therefore can serve as primers for nucleic acid synthesis.

In the PCR, the reaction conditions are cycled between those conducive to hybridization and nucleic acid polymerization, and those which result in the denaturation of duplex molecules. In the first step of the reaction, the nucleic acids of the sample are transiently heated, and then cooled, in order to denature any double-stranded molecules which may be present. The "first" and "second" probes are then added to the sample at a concentration which greatly exceeds that of the desired nucleic acid molecule. When the sample is incubated under conditions conducive to hybridization and polymerization, the "first" probe will hybridize to the nucleic acid molecule of the sample at a position 3' to the sequence to be amplified. If the nucleic acid molecule of the sample was initially double-stranded, the "second" probe will hybridize to the complementary strand of the nucleic acid molecule at a position 3' to the sequence which is the complement of the sequence whose amplification is desired. Upon addition of a polymerase, the 3' ends of the "first" and (if the nucleic acid molecule was double-stranded) "second" probes will be extended. The extension of the "first" probe will result in the synthesis of an oligonucleotide having the exact sequence of the desired nucleic acid. Extension of the "second" probe will result in the synthesis of an oligonucleotide having the exact sequence of the complement of the desired nucleic acid.

The PCR reaction is capable of exponential amplification of specific nucleic acid sequences because the extension product of the "first" probe, of necessity, contains a sequence which is complementary to a sequence of the "second" probe, and thus can serve as a template for the production of an extension product of the "second" probe. Similarly, the extension product of the "second" probe, of necessity, contains a sequence which is complementary to a sequence of the "first" probe, and thus can serve as a template for the production of an extension product of the "first" probe. Thus, by permitting cycles of polymerization, and denaturation, a geometric increase in the concentration of the desired nucleic acid molecule can be achieved. Reviews of the PCR are provided by Mullis, K.B. (Cold Spring Harbor Symp. Quant. Biol. 51:263-273 (1986)); Saiki, R.K., et al. (Bio/Technology 3:1008-1012 (1985)); and Mullis, K.B., et al. (Meth. Enzymol. 155:335-350 (1987)).

In one embodiment, the invention is directed to a naturally occurring mammalian R-PTPase-α. In another embodiment, the invention is directed to a recombinant mammalian R-PTPase-α. The preferred R-PTPases of the present invention are of human origin. The invention provides the naturally occurring molecule substantially free of other proteins with which it is natively associated. "Substantially free of other proteins or glycoproteins" indicates that the protein has been purified away from at least 90 per cent (on a weight basis) and from even at least 99 per cent if desired, of other proteins and glycoproteins with which it is natively associated, and is therefore substantially free of them. That can be achieved by subjecting the cells, tissue or fluids containing the R-PTPase to standard protein purification techniques such as immunoadsorbent columns bearing monoclonal antibodies reactive against the protein. Other forms of affinity purification can utilize solid-phase substrates which can bind the PTPase domain, or a ligand that will bind to the receptor domain. Alternatively, the purification can be achieved by a combination of standard methods, such as ammonium sulfate precipitation, molecular sieve chromatography, and ion exchange chromatography.

It will be understood that the mammalian R-PTPase of the present invention can be biochemically purified from a variety of cell or tissue sources. For preparation of naturally occurring R-PTPase, tissues such as mammalian placenta or brain, especially of human origin, are preferred.

Alternatively, because the gene for the R-PTPase can be isolated or synthesized, the polypeptide can be synthesized substantially free of other proteins or glycoproteins of mammalian origin in a prokaryotic organism or in a non-mammalian eukaryotic organism, if desired. As intended by the present invention, a recombinant R-PTPase-α molecule produced in mammalian cells, such as transfected COS, NIH-3T3, or CHO cells, for example, is either a naturally occurring protein sequence or a functional derivative thereof. Where a naturally occurring protein or glycoprotein is produced by recombinant means, it is provided substantially free of the other proteins and glycoproteins with which it is natively associated.

Alternatively, methods are well known for the synthesis of polypeptides of desired sequence on solid phase supports and their subsequent separation from the support.

In a further embodiment, the invention provides "functional derivatives" of the R-PTPase. By "functional derivative" is meant a "fragment," "variant," "analog," or "chemical derivative" of the R-PTPase, which terms are defined below. A function al derivative retains at least a portion of the function of the R-PTPase, such as binding to a specific antibody, phosphatase enzymatic activity or binding of the extracellular domain to a ligand, which permits its utility in accordance with the present invention.

A "fragment" of the R-PTPase refers to any subset of the molecule, that is, a shorter peptide.

A "variant" of the R-PTPase refers to a molecule substantially similar to either the entire peptide or a fragment thereof. Variant peptides may be conveniently prepared by direct chemical synthesis of the variant peptide, using methods well- known in the art.

Alternatively, amino acid sequence variants of the peptide can be prepared by mutations in the DNA which encodes the synthesized peptide. Such variants include, for example, deletions from, or insertions or substitutions of, residues within the amino acid sequence. Any combination of deletion, insertion, and substitution may also be made to arrive at the final construct, provided that the final construct possesses the desired activity. Obviously, the mutations that will be made in the DNA encoding the variant peptide must not alter the reading frame and preferably will not create complementary regions that could produce secondary mRNA structure (see European Patent Publication No. EP 75,444).

At the genetic level, these variants ordinarily are prepared by site-directed mutagenesis (as exemplified by Adelman et al., DNA 2:183 (1983)) of nucleotides in the DNA encoding the peptide molecule, thereby producing DNA encoding the variant, and thereafter expressing the DNA in recombinant cell culture (see below). The variants typically exhibit the same qualitative biological activity as the nonvariant peptide.

An "analog" of the R-PTPase refers to a non-natural molecule substantially similar to either the entire molecule or a fragment thereof.

A "chemical derivative" of the R-PTPase contains additional chemical moieties not normally a part of the peptide. Covalent modifications of the peptide are included within the scope of this invention. Such modifications may be introduced into the molecule by reacting targeted amino acid residues of the peptide with an organic derivatizing agent that is capable of reacting with selected side chains or terminal residues.

Cysteinyl residues most commonly are reacted with alpha-haloacetates (and corresponding amines), such as chloroacetic acid or chloroacetamide, to give carboxymethyl or carboxyamidomethyl derivatives. Cysteinyl residues also are derivatized by reaction with bromotrifluoroacetone, alpha-bromo- beta-(5-imidozoyl)propionic acid, chloroacetyl phosphate, N- alkylmaleimides, 3-nitro-2-pyridyl disulfide, methyl 2-pyridyl disulfide, p-chloromercuribenzoate, 2-chloromercuri-4- nitrophenol, or chloro-7-nitrobenzo-2-oxa-1,3-diazole.

Histidyl residues are derivatized by reaction with diethylprocarbonate at pH 5.5-7.0 because this agent is relatively specific for the histidyl side chain. Para-bromophenacyl bromide also is useful; the reaction is preferably performed in 0.1 M sodium cacodylate at pH 6.0.

Lysinyl and amino terminal residues are reacted with succinic or other carboxylic acid anhydrides. Derivatization with these agents has the effect of reversing the charge of the lysinyl residues. Other suitable reagents for derivatizing alpha-amino-containing residues include imidoesters such as methyl picolinimidate; pyridoxal phosphate; pyridoxal; chloroborohydride; trinitrobenzenesulfonic acid; O-methylisourea; 2,4 pentanedione; and transaminase-catalyzed reaction with glyoxylate.

Arginyl residues are modified by reaction with one or several conventional reagents, among them phenylglyoxal, 2,3- butanedione, 1,2-cyclohexanedione, and ninhydrin. Derivatization of arginine residues requires that the reaction be performed in alkaline conditions because of the high pKₐ of the guanidine functional group. Furthermore, these reagents may react with the groups of lysine as well as the arginine epsilon-amino group.

The specific modification of tyrosyl residues per se has been studied extensively, with particular interest in introducing spectral labels into tyrosyl residues by reaction with aromatic diazonium compounds or tetranitromethane. Most commonly, N-acetylimidizol and tetranitromethane are used to form O-acetyl tyrosyl species and 3-nitro derivatives, respectively.

Carboxyl side groups (aspartyl or glutamyl) are selectively modified by reaction with carbodiimides (R'-N-C-N-R') such as 1-cyclohexyl-3-(2-morpholinyl-(4-ethyl) carbodiimide or 1- ethyl-3- (4-azonia-4,4-dimethylpentyl) carbodiimide. Furthermore, aspartyl and glutamyl residues are converted to asparaginyl and glutaminyl residues by reaction with ammonium ions.

Glutaminyl and asparaginyl residues are frequently deamidated to the corresponding glutamyl and aspartyl residues. Alternatively, these residues are deamidated under mildly acidic conditions. Either form of these residues falls within the scope of this invention.

Derivatization with bifunctional agents is useful for cross-linking the peptide to a water-insoluble support matrix or to other macromolecular carriers. Commonly used cross-linking agents include, e.g., 11-bis(diazoacetyl)-2-phenylethane, glutaraldehyde, N-hydroxysuccinimide esters, for example, esters with 4-azidosalicylic acid, homobifunctional imidoesters, including disuccinimidyl esters such as 3,3'- dithiobis(succinimidyl-propionate), and bifunctional maleimides such as bis-N-maleimido-1,8-octane. Derivatizing agents such as methyl-3-[(p-azidophenyl)dithio]propioimidate yield photoactivatable intermediates that are capable of forming crosslinks in the presence of light. Alternatively, reactive water-insoluble matrices such as cyanogen bromide-activated carbo hydrates and the reactive substrates described in U.S. Patent Nos. 3,969,287+ 3,691,016; 4,195,128; 4,247,642; 4,229,537; and 4,330,440 are employed for protein immobilization.

Other modifications include hydroxylation of proline and lysine, phosphorylation of hydroxyl groups of seryl or threonyl residues, methylation of the alpha-amino groups of lysine, arginine, and histidine side chains (T.E. Creighton, Proteins: Structure and Molecule Properties, W.H. Freeman & Co., San Francisco, pp. 79-86 (1983)), acetylation of the N-terminal amine, and, in some instances, amidation of the C-terminal carboxyl groups.

Such derivatized moieties may improve the solubility, absorption, biological half life, and the like. The moieties may alternatively eliminate or attenuate any undesirable side effect of the protein and the like. Moieties capable of mediating such effects are disclosed, for example, in Remington's Pharmaceutical Sciences, 16th ed., Mack Publishing Co., Easton, PA (1980)

This invention is also directed to an antibody specific for an epitope of R-PTPase, preferably, of R-PTPase-α, most preferably of human R-PTPase-α, and the use of such antibody to detect the presence of, or measure the quantity or concentration of, the R-PTPase in a cell, a cell or tissue extract, or a biological fluid.

The term "antibody" is meant to include polyclonal antibodies, monoclonal antibodies (mAbs), chimeric antibodies, and anti-idiotypic (anti-Id) antibodies.

Polyclonal antibodies are heterogeneous populations of antibody molecules derived from the sera of animals immunized with an antigen.

Monoclonal antibodies are a substantially homogeneous population of antibodies to specific antigens. MAbs may be obtained by methods known to those skilled in the art. See, for example Kohler and Milstein, Nature 256:495-497 (1975) and U.S. Patent No. 4,376,110. Such antibodies may be of any immunoglobulin class including IgG, IgM, IgE, IgA, GILD and any subclass thereof. The hybridoma producing the mAbs of this invention may be cultivated in vitro or in vivo. Production of high titers of mAbs in vivo production makes this the presently preferred method of production. Briefly, cells from the individual hybridomas are injected intraperitoneally into pristane-primed BALB/c mice to produce ascites fluid containing high concentrations of the desired mAbs. MAbs of isotype IgM or IgG may be purified from such ascites fluids, or from culture supernatants, using column chromatography methods well known to those of skill in the art.

Chimeric antibodies are molecules containing different portions of that are derived from different animal species such as those having variable region derived from a murine mAb and a human immunoglobulin constant region. Chimeric antibodies and methods for their production are known in the art ( Cabilly et al, Proc. Natl. Acad. Sci. USA 81:3273-3277 (1984); Morrison et al., Proc. Natl. Acad. Sci. USA 81:6851-6855 (1984); Boulianne et al., Nature 312:643-646 (1984); Cabilly et al., European Patent Application 125023 (published November 14, 1984); Neuberger et al., Nature 314:268-270 (1985); Taniguchi et al., European Patent Application 171496 (published February 19, 1985); Morrison et al., European Patent Application 173494 (published March 5, 1986); Neuberger et al., PCT Application WO 86/01533 (published March 13, 1986); Kudo et al., European Patent Application 184187 (published June 11, 1986); Morrison et al., European Patent Application 173494 (published March 5, 1986); Sahagan et al., J. Immunol. 137:1066-1074 (1986); Robinson et al., International Patent Publication #PCT/US86/02269 (published 7 May 1987); Liu et al., Proc. Natl. Acad. Sci. USA 84:3439-3443 (1987); Sun et al., Proc. Natl. Acad. Sci. USA 84:214-218 (1987); Better et al., Science 240:1041- 1043 (1988)). These references are hereby incorporated by reference.

An anti-idiotypic (anti-Id) antibody is an antibody which recognizes unique determinants generally associated with the antigen-binding site of an antibody. An anti-Id antibody can be prepared by immunizing an animal of the same species and genetic type (e.g. mouse strain) as the source of the mAb with the mAb to which an anti-Id is being prepared. The immunized animal will recognize and respond to the idiotypic determinants of the immunizing antibody by producing an antibody to these idiotypic determinants (the anti-Id antibody).

The anti-Id antibody may also be used as an "immunogen" to induce an immune response in yet another animal, producing a so-called anti-anti-Id antibody. The anti-anti-Id may be epitopically identical to the original mAb which induced the anti-Id. Thus, by using antibodies to the idiotypic determinants of a mAb, it is possible to identify other clones expressing antibodies of identical specificity.

Accordingly, mAbs generated against the R-PTPase of the present invention may be used to induce anti-Id antibodies in suitable animals, such as BALB/c mice. Spleen cells from such immunized mice are used to produce anti-Id hybridomas secreting anti-Id mAbs. Further, the anti-Id 5 mAbs can be coupled to a carrier such as keyhole limpet hemocyanin (KLH) and used to immunize additional BALB/c mice. Sera from these mice will contain anti-anti-Id antibodies that have the binding properties of the original mAb specific for a R-PTPase epitope.

The anti-Id mAbs thus have their own idiotypic epitopes, or "idiotopes" structurally similar to the epitope being evaluated, such as R-PTPase-α.

The term "antibody" is also meant to include both intact molecules as well as fragments thereof, such as, for example, Fab and F(ab')₂, which are capable of binding antigen. Fab and F(ab')₂ fragments lack the Fc fragment of intact antibody, clear more rapidly from the circulation, and may have less non-specific tissue binding than an intact antibody (Wahl et al., J. Nucl. Med. 24:316-325 (1983)).

It will be appreciated that Fab and F(ab')₂ and other fragments of the antibodies useful in the present invention may be used for the detection and quantitation of R-PTPase according to the methods disclosed herein for intact antibody molecules. . Such fragments are typically produced by proteolytic cleavage, using enzymes such as papain (to produce Fab fragments) or pepsin (to produce F(ab')₂ fragments).

An antibody is said to be "capable of binding" a molecule if it is capable of specifically reacting with the molecule to thereby bind the molecule to the antibody. The term "epitope" is meant to refer to that portion of any molecule capable of being bound by an antibody which can also be recognized by that antibody. Epitopes or "antigenic determinants" usually consist of chemically active surface groupings of molecules such as amino acids or sugar side chains and have specific three dimensional structural characteristics as well as specific charge characteristics.

An "antigen" is a molecule or a portion of a molecule capable of being bound by an antibody which is additionally capable of inducing an animal to produce antibody capable of binding to an epitope of that antigen. An antigen may have one, or more than one epitope. The specific reaction referred to above is meant to indicate that the antigen will react, in a highly selective manner, with its corresponding antibody and not with the multitude of other antibodies which may be evoked by other antigens.

The antibodies, or fragments of antibodies, useful in the present invention may be used to quantitatively or qualitatively detect the presence of cells which express the R-PTPase protein. This can be accomplished by immunofluorescence techniques employing a fluorescently labeled antibody (see below) coupled with light microscopic, flow cytometric, or fluorimetric detection.

The antibodies (of fragments thereof) useful in the present invention may be employed histologically, as in immunofluorescence or immunoelectron microscopy, for in situ detection of R-PTPase. In situ detection may be accomplished by removing a histological specimen from a patient, and providing a labeled antibody of the present invention to such a specimen. The antibody (or fragment) is preferably provided by applying or by overlaying the labeled antibody (or fragment) to a biological sample. Through the use of such a procedure, it is possible to determine not only the presence of the R-PTPase but also its distribution on the examined tissue. Using the present invention, those of ordinary skill will readily perceive that any of a wide variety of histological methods (such as staining procedures) can be modified in order to achieve such in situ detection. Such assays for R-PTPase typically comprise incubating a biological sample, such as a biological fluid, a tissue extract, freshly harvested cells such as lymphocytes or leucocytes, or cells which have been incubated in tissue culture, in the presence of a detectably labeled antibody capable of identifying R-PTPase, and detecting the antibody by any of a number of techniques well-known in the art.

The biological sample may be treated with a solid phase support such as nitrocellulose, or other solid support which is capable of immobilizing cells, cell particles or soluble proteins. The support may then be washed with suitable buffers followed by treatment with the detectably labeled R-PTPase-specific antibody. The solid phase support may then be washed with the buffer a second time to remove unbound antibody. The amount of bound label on said solid support may then be detected by conventional means.

By "solid phase support" is intended any support capable of binding antigen or antibodies. Well-known supports, or carriers, include glass, polystyrene, polypropylene, polyethylene, dextran, nylon, amylases, natural and modified celluloses, polyacrylamides, gabbros, and magnetite. The nature of the carrier can be either soluble to some extent or insoluble for the purposes of the present invention. The support material may have virtually any possible structural configuration so long as the coupled molecule is capable of binding to an antigen or antibody. Thus, the support configuration may be spherical, as in a bead, or cylindrical, as in the inside surface of a test tube, or the external surface of a rod. Alternatively, the surface may be flat such as a sheet, test strip, etc. Preferred supports include polystyrene beads. Those skilled in the art will know many other suitable carriers for binding antibody or antigen, or will be able to ascertain the same by use of routine experimentation.

The binding activity of a given lot of anti-R-PTPase antibody may be determined according to well known methods. Those skilled in the art will be able to determine operative and optimal assay conditions for each determination by employing routine experimentation.

Other such steps as washing, stirring, shaking, filtering and the like may be added to the assays as is customary or necessary for the particular situation.

One of the ways in which the R-PTPase-specific antibody can be detectably labeled is by linking the same to an enzyme and use in an enzyme immunoassay (EIA). This enzyme, in turn, when later exposed to an appropriate substrate, will react with the substrate in such a manner as to produce a chemical moiety which can be detected, for example, by spectrophotometric, fluorimetric or by visual means. Enzymes which can be used to detectably label the antibody include, but are not limited to, malate dehydrogenase, staphylococcal nuclease, delta-5-steroid isomerase, yeast alcohol dehydrogenase, alpha-glycerophosphate dehydrogenase, triose phosphate isomerase, horseradish peroxidase, alkaline phosphatase, asparaginase, glucose oxidase, beta-galactosidase, ribonuclease, urease, catalase, glucose-6-phosphate dehydrogenase, glucoamylase and acetylcholinesterase. The detection can be accomplished by colorimetric methods which employ a chromogenic substrate for the enzyme. Detection may also be accomplished by visual comparison of the extent of enzymatic reaction of a substrate in comparison with similarly prepared standards.

Detection may be accomplished using any of a variety of other immunoassays. For example, by radioactively labeling the antibodies or antibody fragments, it is possible to detect R-PTPase through the use of a radioimmunoassay (RIA) (see, for example, Work, T.S. et al., Laboratory Techniques and Biochemistry in Molecular Biology, North Holland Publishing Company, New York, 1978, which is incorporated by reference herein). The radioactive isotope can be detected by such means as the use of a gamma counter or a scintillation counter or by autoradiography.

It is also possible to label the antibody with a fluorescent compound. When the fluorescently labeled antibody is exposed to light of the proper wave length, its presence can then be detected due to fluorescence. Among the most commonly used fluorescent labelling compounds are fluorescein isothiocyanate, rhodamine, phycoerythrin, phycocyanin, allophycocyanin, o- phthaldehyde and fluorescamine.

The antibody can also be detectably labeled using fluorescence emitting metals such as ¹⁵²Eu, or others of the lanthanide series. These metals can be attached to the antibody using such metal chelating groups as diethylenetriaminepentaacetic acid (DTPA) or ethylenediaminetetraacetic acid (EDTA).

The antibody also can be detectably labeled by coupling it to a chemiluminescent compound. The presence of the chemiluminescent-tagged antibody is then determined by detecting the presence of luminescence that arises during the course of a chemical reaction. Examples of particularly useful chemiluminescent labeling compounds are luminol, isoluminol, theromatic acridinium ester, imidazole, acridinium salt and oxalate ester.

Likewise, a bioluminescent compound may be used to label the antibody of the present invention. Bioluminescence is a type of chemiluminescence found in biological systems in which a catalytic protein increases the efficiency of the chemiluminescent reaction. The presence of a bioluminescent protein is determined by detecting the presence of luminescence. Important bioluminescent compounds for purposes of labeling are luciferin, luciferase and aequorin.

The antibody molecules of the present invention may be adapted for utilization in an mmunometric assay, also known as a "two-site" or "sandwich" assay. In a typical immunometric assay, a quantity of unlabeled antibody (or fragment of antibody) is bound to a solid support and a quantity of detectably labeled soluble antibody is added to permit detection and/or quantitation of the ternary complex formed between solid-phase antibody, antigen, and labeled antibody.

Typical, and preferred, immunometric assays include "forward" assays in which the antibody bound to the solid phase is first contacted with the sample being tested to extract the antigen from the sample by formation of a binary solid phase antibody-antigen complex. After a suitable incubation period, the solid support is washed to remove the residue of the fluid sample, including unreacted antigen, if any, and then contacted with the solution containing an unknown quantity of labeled antibody (which functions as a "reporter molecule"). After a second incubation period to permit the labeled antibody to complex with the antigen bound to the solid support through the unlabeled antibody, the solid support is washed a second time to remove the unreacted labeled antibody.

In another type of "sandwich" assay, which may also be useful with the antigens of the present invention, the so-called "simultaneous" and "reverse" assays are used. A simultaneous assay involves a single incubation step as the antibody bound to the solid support and labeled antibody are both added to the sample being tested at the same time. After the incubation is completed, the solid support is washed to remove the residue of fluid sample and uncomplexed labeled antibody. The presence of labeled antibody associated with the solid support is then determined as it would be in a conventional "forward" sandwich assay.

In the "reverse" assay, stepwise addition first of a solution of labeled antibody to the fluid sample followed by the addition of unlabeled antibody bound to a solid support after a suitable incubation period is utilized After a second incubation, the solid phase is washed in conventional fashion to free it of the residue of the sample being tested and the solution of unreacted labeled antibody. The determination of labeled antibody associated with a solid support is then determined as in the "simultaneous" and "forward" assays.

The presence of normally functioning R-PTPase in a subject can also be tested using direct enzymatic assays, for the tyrosine phosphatase activity. Such biochemical measurements can be performed in vitro, using purified enzymes, allowing precise measurements of enzyme activity, or with membrane preparations, or whole cells, where the net phosphotyrosine level is determined.

In additional embodiments of the present invention, a DNA sequence encoding a R-PTPase molecule and methods for expressing the DNA sequence are provided. One of ordinary skill in the art will know how to identify and clone additional PTPase molecules, of human or other mammalian species, which have sequence homology to the R-PTPase molecules described herein, using the genetic sequences and oligonucleotides of the present invention without undue experimentation. Furthermore, manipulation of the genetic constructs of the present invention allow the grafting of a particular ligand-binding receptor domain onto the transmembrane and catalytic portions of the R-PTPase resulting in chimeric molecules. Non-limiting examples of such chimeric molecules include the R-PTPase wherein the receptor is an epidermal growth factor receptor, a fibroblast growth factor receptor, and the like.
Genetically engineered chimeric receptors are known in the art (see, for example, Riedel, H. et al., Nature 324:628-670 (1986)).

Genetic constructs encoding R-PTPase-α, functional derivative thereof, and chimeric molecules such as those described above, can be used in gene therapy. An abnormal or dysfunctional R-PTPase, which results in disease, may be replaced by infusion of cells of the desired lineage (such as hemopoietic cells, for example) transfected with a normal R-PTPase. Alternatively, or additionally, cells carrying a chimeric R-PTPase having a receptor to a ligand of choice (e.g. EGF) can be used for such gene therapy.

The recombinant DNA molecules of the present invention can be produced through any of a variety of means, such as, for example, DNA or RNA synthesis, or more preferably, by application of recombinant DNA techniques. Techniques for synthesizing such molecules are disclosed by, for example, Wu, R., et al. (Prog. Nucl. Acid. Res. Molec. Biol. 21:101-141 (1978)). Procedures for constructing recombinant molecules in accordance with the above-described method are disclosed by Sambrook et al. (supra).

The 3' terminus of the recombinant molecule of this invention is preferably treated to render it unsuitable for polymerization. Such treatment may be accomplished by blocking the terminus by chemical means, or by modifying the terminal bases such that they sterically interfere with polymerase action. In a preferred embodiment, such treatment is accomplished by immobilizing the 3' terminus, such as by coupling it to a solid support (such as, for example, glass, plastic, latex, etc.). The support may be of any form (i.e. a sheet, rod, sphere, ovoid, etc. Procedures for such immobilization are well known to those of ordinary skill. In the most preferred embodiment, the 3' end of the recombinant molecule is covalently bound to the solid support. A spacer region may be used to extend the probe outward from the solid support as long as (1) it will not sterically hinder any function or characteristic of the recombinant molecule, and (2) the sequence of the spacer region does not participate in the hybridization or polymerization reactions of the assay. It is typically desirable to immobilize several, and preferably, a large number of such recombinant molecule to the support.

Oligonucleotides representing a portion of an R-PTPase are useful for screening for the presence of genes encoding such proteins and for the cloning of R-PTPase genes. Techniques for synthesizing such oligonucleotides are disclosed by, for example, Wu, R., et al., Prog. Nucl. Acid. Res. Molec. Biol. 21:101-141 (1978)).

Protein molecules are fragmented as with cyanogen bromide, or with proteases such as papain, chymotrypsin, trypsin, etc. (Oike, Y., et al., J. Biol. Chem. 257:9751-9758 (1982); Liu, C., et al., Int. J. Pent. Protein Res. 21:209-215 (1983)). Because the genetic code is degenerate, more than one codon may be used to encode a particular amino acid (Watson, J.D., In: Molecular Biology of the Gene, 4th Ed., Benjamin/Cummings Publishing Co., Inc., Menlo Park, CA (1987)). Using the genetic code, one or more different oligonucleotides can be identified, each of which would be capable of encoding the amino acid. The probability that a particular oligonucleotide will, in fact, constitute the actual XXX-encoding sequence can be estimated by considering abnormal base pairing relationships and the frequency with which a particular codon is actually used (to encode a particular amino acid) in eukaryotic cells. Such "codon usage rules" are disclosed by Lathe, R., et al., J. Molec. Biol. 183:1-12 (1985). Using the "codon usage rules" of Lathe, a single oligonucleotide, or a set of oligonucleotides, that contains a theoretical "most probable" nucleotide sequence capable of encoding the R-PTPase sequences is identified.

Although occasionally an amino acid sequence may be encoded by only a single oligonucleotide, frequently the amino acid sequence may be encoded by any of a set of similar oligonucleotides. Importantly, whereas all of the members of this set contain oligonucleotides which are capable of encoding the peptide fragment and, thus, potentially contain the same oligonucleotide sequence as the gene which encodes the peptide fragment, only one member of the set contains the nucleotide sequence that is identical to the nucleotide sequence of the gene. Because this member is present within the set, and is capable of hybridizing to DNA even in the presence of the other members of the set, it is possible to employ the unfractionated set of oligonucleotides in the same manner in which one would employ a single oligonucleotide to clone the gene that encodes the peptide.

The oligonucleotide, or set of oligonucleotides, containing the theoretical "most probable" sequence capable of encoding the R-PTPase fragment is used to identify the sequence of a complementary oligonucleotide or set of oligonucleotides which is capable of hybridizing to the "most probable" sequence, or set of sequences. An oligonucleotide containing such a complementary sequence can be employed as a probe to identify and isolate the R-PTPase gene (Sambrook et al., supra).

A suitable oligonucleotide, or set of oligonucleotides, which is capable of encoding a fragment of the R-PTPase gene (or which is complementary to such an oligonucleotide, or set of oligonucleotides) is identified (using the above-described procedure), synthesized, and hybridized by means well known in the art, against a DNA or, more preferably, a cDNA preparation derived from cells which are capable of expressing the R-PTPase gene. Single stranded oligonucleotide molecules complementary to the "most probable" R-PTPase peptide encoding sequences can be synthesized using procedures which are well known to those of ordinary skill in the art (Belagaje, R., et al., J. Biol. Chem. 254:5765-5780 (1979); Maniatis, T., et al., In: Molecular Mechanisms in the Control of Gene Expression, Nierlich, D.P., et al., Eds., Acad. Press, NY (1976); Wu, R., et al., Prog. Nucl. Acid Res. Molec. Biol. 21:101-141 (1978); Khorana, R.G., Science 203:614-625 (1979)).
Additionally, DNA synthesis may be achieved through the use of automated synthesizers. Techniques of nucleic acid hybridization are disclosed by Sambrook et al. (supra), and by Haymes, B.D., et al. (In: Nucleic Acid Hybridization, A Practical Approach, IRL Press, Washington, DC (1985)), which references are herein incorporated by reference. Techniques such as, or similar to, those described above have successfully enabled the cloning of genes for human aldehyde dehydrogenases (Hsu, L.C., et al., Proc. Natl. Acad. Sci. USA 82:3771-3775 (1985)), fibronectin (Suzuki, S., et al., EMBO J. 4:2519-2524 (1985)), the human estrogen receptor gene (Walter, P., et al., Proc. Natl. Acad. Sci. USA 82:7889-7893 (1985)), tissue-type plasminogen activator (Pennica, D., et al., Nature 301:214-221 (1983)) and human term placental alkaline phosphatase complementary DNA (Kam, W., et al., Proc. Natl. Acad. Sci. USA 82:(715-8719 (1985)).

In a alternative way of cloning the R-PTPase gene, a library of expression vectors is prepared by cloning DNA or, more preferably, cDNA (from a cell capable of expressing R-PTPase) into an expression vector. The library is then screened for members capable of expressing a protein which binds to anti-R-PTPase antibody, and which has a nucleotide sequence that is capable of encoding polypeptides that have the same amino acid sequence as R-PTPase, or fragments thereof. In this embodiment, DNA, or more preferably cDNA, is extracted and purified from a cell which is capable of expressing R-PTPase protein. The purified cDNA is fragmented (by shearing, endonuclease digestion, etc.) to produce a pool of DNA or cDNA fragments. DNA or cDNA fragments from this pool are then cloned into an expression vector in order to produce a genomic library of expression vectors whose members each contain a unique cloned DNA or cDNA fragment.

An "expression vector" is a vector which (due to the presence of appropriate transcriptional and/or translational control sequences) is capable of expressing a DNA (or cDNA) molecule which has been cloned into the vector and of thereby producing a polypeptide or protein.
Expression of the cloned sequences occurs when the expression vector is introduced into an appropriate host cell. If a prokaryotic expression vector is employed, then the appropriate host cell would be any prokaryotic cell capable of expressing the cloned sequences. Similarly, if a eukaryotic expression vector is employed, then the appropriate host cell would be any eukaryotic cell capable of expressing the cloned sequences. Importantly, since eukaryotic DNA may contain intervening sequences, and since such sequences cannot be correct ly processed in prokaryotic cells, it is preferable to employ cDNA from a cell which is capable of expressing R-PTPase in order to produce a prokaryotic genomic expression vector library. Procedures for preparing cDNA and for producing a genomic library are disclosed by Sambrook et al. (supra).

A DNA sequence encoding the R-PTPase of the present invention, or its functional derivatives, may be recombined with vector DNA in accordance with conventional techniques, including blunt-ended or staggered-ended termini for ligation, restriction enzyme digestion to provide appropriate termini, filling in of cohesive ends as appropriate, alkaline phosphatase treatment to avoid undesirable joining, and ligation with appropriate ligases. Techniques for such manipulations are disclosed by Sambrook et al., supra, and are well known in the art.

A nucleic acid molecule, such as DNA, is said to be "capable of expressing" a polypeptide if it contains nucleotide sequences which contain transcriptional and translational regulatory information and such sequences are "operably linked" to nucleotide sequences which encode the polypeptide. An operable linkage is a linkage in which the regulatory DNA sequences and the DNA sequence sought to be expressed are connected in such a way as to permit gene expression. The precise nature of the regulatory regions needed for gene expression may vary from organism to organism, but shall in general include a promoter region which, in prokaryotes, contains both the promoter (which directs the initiation of RNA transcription) as well as the DNA sequences which, when transcribed into RNA, will signal the initiation of protein synthesis. Such regions will normally include those 5'-non-coding sequences involved with initiation of transcription and translation, such as the TATA box, capping sequence, CAAT sequence, and the like.

If desired, the non-coding region 3' to the gene sequence coding for the protein may be obtained by the above-described methods. This region may be retained for its transcriptional termination regulatory sequences, such as termination and polyadenylation. Thus, by retaining the 3'-region naturally contiguous to the DNA sequence coding for the protein, the transcriptional termination signals may be provided. Where the transcriptional termination signals are not satisfactorily functional in the expression host cell, then a 3' region functional in the host cell may be substituted.

Two DNA sequences (such as a promoter region sequence and a R-PTPase-encoding sequence) are said to be operably linked if the nature of the linkage between the two DNA sequences does not (1) result in the introduction of a frame-shift mutation, (2) interfere with the ability of the promoter region sequence to direct the transcription of the R-PTPase gene sequence, or (3) interfere with the ability of the R-PTPase gene sequence to be transcribed by the promoter region sequence. A promoter region would be operably linked to a DNA sequence if the promoter were capable of effecting transcription of that DNA sequence. Thus, to express the protein, transcriptional and translational signals recognized by an appropriate host are necessary.

A promoter is a double-stranded DNA or RNA molecule which is capable of binding RNA polymerase and promoting the transcription of an "operably linked" nucleic acid sequence. As used herein, a "promoter sequence" is the sequence of the promoter which is found on that strand of the DNA or RNA which is transcribed by the RNA polymerase. A "promoter sequence complement" is a nucleic acid molecule whose sequence is the complement of a "promoter sequence." Hence, upon extension of a primer DNA or RNA adjacent to a single-stranded "promoter sequence complement" or, of a "promoter sequence," a double-stranded molecule is created which will contain a functional promoter, if that extension proceeds towards the "promoter sequence" or the "promoter sequence complement." This functional promoter will direct the transcription of a nucleic acid molecule which is operably linked to that strand of the double-stranded molecule which contains the "promoter sequence" (and not that strand of the molecule which contains the "promoter sequence complement").

Certain RNA polymerases exhibit a high specificity for such promoters. The RNA polymerases of the bacteriophages T7, T3, and SP-6 are especially well characterized, and exhibit high promoter specificity. The promoter sequences which are specific for each of these RNA polymerases also direct the polymerase to utilize (i.e. transcribe) only one strand of the two strands of a duplex DNA template. The selection of which strand is transcribed is determined by the orientation of the promoter sequence. This selection determines the direction of transcription since RNA is only polymerized enzymatically by the addition of a nucleotide 5' phosphate to a 3' hydroxyl terminus.

Two sequences of a nucleic acid molecule are said to be "operably linked" when they are linked to each other in a manner which either permits both sequences to be transcribed onto the same RNA transcript, or permits an RNA transcript, begun in one sequence to be extended into the second sequence. Thus, two sequences, such as a promoter sequence and any other "second" sequence of DNA or RNA are operably linked if transcription commencing in the promoter sequence will produce an RNA transcript of the operably linked second sequence. In order to be "operably linked" it is not necessary that two sequences be immediately adjacent to one another.

Thus, as indicated above, in order to function as a promoter, a promoter sequence must be present as a double-stranded molecule. For the purposes of the present invention, the two strands of a functional promoter sequence are referred to as a "transcript" strand and a "complementary" strand. The "transcript" strand is that strand of the duplex which will be transcribed by the RNA polymerase (i.e. which serves as the template for transcription). The "complementary" strand is the strand which has a sequence complementary to the "transcript" strand, and which must be present, and hybridized to the "transcript" strand, in order for transcription to occur. Thus, when the "transcript" strand of a promoter sequence is operably linked to a second sequence, hybridization of the "transcript" strand with the "complement" strand, will, in the presence of a polymerase, result in the transcription of the "transcript" strand, and will produce an RNA transcript using the sequence of the "transcript" strand as a template.

The promoter sequences of the present invention may be either prokaryotic, eukaryotic or viral. Suitable promoters are repressible, or, more preferably, constitutive. Examples of suitable prokaryotic promoters include promoters capable of recognizing the T4 (Malik, S. et al., J. Biol. Chem. 263:1174-1181 (1984); Rosenberg, A.H. et al., Gene 59:191-200 (1987); Shinedling, S. et al., J. Molec. Biol. 195:471-480 (1987); Hu, M. et al., Gene 42:21-30 (1986)), T3, Sp6, and T7 (Chamberlin, M. et al. , Nature 228:227-231 (1970); Bailey, J.N. et al., Proc. Natl. Acad. Sci. (U.S.A.) 80:2814-2818 (1983); Davanloo, P. et al., Proc. Natl. Acad. Sci. (U.S.A.) 81:2035-2039 (1984)) polymerases; the P_{R} and P_{L} promoters of bacteriophage lambda (The Bacteriophage Lambda, Hershey, A.D., Ed., Cold Spring Harbor Press, Cold Spring Harbor, NY (1973); Lambda II, Hendrix, R.W., Ed., Cold Spring Harbor Press, Cold Spring Harbor, NY (1980)); the trp, recA, heat shock, and lacZ promoters of E. coli; the α-amylase (Ulmanen, I., et al., J. Bacteriol. 162:176-182 (1985)) and the σ-28-specific promoters of B. subtilis (Gilman, M.Z., et al., Gene 32:11-20 (1984)); the promoters of the bacteriophages of Bacillus (Gryczan, T.J., In: The Molecular Biology of the Bacilli, Academic Press, Inc., NY (1982)); Streptomyces promoters (Ward, J.M., et al., Mol. Gen. Genet. 203:468-478 (1986)); the int promoter of bacteriophage lambda; the bla promoter of the β-lactamase gene of pBR322, and the CAT promoter of the chloramphenicol acetyl transferase gene of pPR325, etc. Prokaryotic promoters are reviewed by Glick, B.R. (J. Ind. Microbiol. 1:277-282 (1987)); Cenatiempo, Y. (Biochimie 68:505-516 (1986)); Watson, J.D. et al. (In: Molecular Biology of the Gene, Fourth Edition, Benjamin Cummins, Menlo Park, CA (1987)); and Gottesman, S. (Ann. Rev. Genet. 18:415-442 (1984)). Preferred eukaryotic promoters include the promoter of the mouse metallothionein I gene (Hamer, D., et al., J. Mol. Appl. Gen. 1:273-288 (1982)); the TK promoter of Herpes virus (McKnight, S., Cell 31:355-365 (1982)); the SV40 early promoter (Benoist, C., et al., Nature (London) 290:304-310 (1981)); and the yeast gal4 gene promoter (Johnston, S.A., et al., Proc. Natl. Acad. Sci. (USA) 79:6971-6975 (1982); Silver, P.A., et al., Proc. Natl. Acad. Sci. (USA) 81:5951-5955 (1984)). All of the above listed references are incorporated by reference herein.

Strong promoters are preferred. Examples of such preferred promoters are those which recognize the T3, SP6 and T7 polymerases, the P_{L} promoter of bacteriophage lambda, the recA promoter and the promoter of the mouse metallothionein I gene. A most preferred promoter for eukaryotic expression of R-PTPase is an SV40 promoter such as that driving transcription in the pLSV vector (Livneh, E., et al., (1986) J. Biol. Chem. 261, 12490- 12497). The sequences of such polymerase recognition sites are disclosed by Watson, J.D. et al. (In: Molecular Biology of the Gene, Fourth Edition, Benjamin/Cummings Publishing Co., Inc., Menlo Park, CA, (1987)).

Having now generally described the invention, the same will be more readily understood through reference to the following examples which are provided by way of illustration, and are not intended to be limiting of the present invention, unless specified.

### EXAMPLE I

### Isolation and Analysis of Murine R-PTPase-α cDNA Clones

### 1. Library screening

A mouse BALB/C brain cDNA library in lambda gtll (obtained from Dr. Y. Citri) was screened at relaxed stringency (6XSSC, 5XDenhardts, 0.1% SDS, 50 mM Tris pH 7.5, 1 mM EDTA, 0.1 mg/ml salmon sperm DNA, hybridization temperature 50°C) using as a probe a 2400 bp BglII-AccI fragment representing the intracellular and trans-membrane domains of the human T200 glycoprotein (Ralph, S.J. et al., (1987) EMBO J. 6, 1251-1257), which had been ³²P-labeled using the random-priming method. Washing was performed at 50°C in 6XSSC, 0.1%SDS. Out of 10⁶ clones, 51 positives were picked, selected and characterized by restriction enzyme mapping. EcoRI fragments of 0.95, 1.6 and 0.3 Kb isolated from the phage clone containing the longest insert (lambda-109) were subcloned into the Bluescript KS plus and minus vectors. A series of nested deletions were generated by taking use of restriction sites common to the cloned cDNA fragments and the polylinker region of the plasmid vector. The individual restriction sites used are indicated in Figure 1b. Single stranded DNA was prepared from these constructs, and used as a template for sequence analysis using the dideoxynucleotide chain termination method (Sequenase, United States Biochemical). All regions were sequenced on both strands. The relative order and orientation of the EcoRI fragments in the recombinant phage was determined by restriction mapping. To ascertain that the different EcoRI fragments did not correspond to unrelated cDNA fragments ligated together during the process of library construction, restriction mapping was also performed on a different and independent isolate, lambda-113.

### 2. Results

Brain tissue already has proven to be a rich source of many types of tyrosine kinases, and recent biochemical evidence has also indicated the existence of multiple forms of PTPase activity (Jones, S.W. et al., (1989) J. Biol. Chem. 264, 7747-7753). In order to search for new receptor-type PTPase, the present inventors screened at low stringency a mouse brain cDNA library, using as a hybridization probe the intracellular domain of human CD45 containing two tandem PTPase domains (Tonks, N.K. et al., supra; Charbonneau, H. et al., supra; Ralph, S.J. et al., supra). Positive clones were classified by cross-hybridization and restriction mapping into several categories, and the longest phage insert (lambda-109) corresponding to the most abundantly represented class was chosen for subcloning and further analysis.

The result of the nucleotide sequence analysis is shown in Figure 1. Conceptual translation of the cDNA sequence reveals the existence of a major open reading frame of 794 amino acids, assuming that translation initiates at nucleotide 259 (an in-frame stop codon is present 60 nucleotides upstream). The putative initiation methionine codon is embedded in a relatively standard environment for initiation of translation (Kozak, M., (1987) Nucl. Ac. Res. 15, 8125-8148), and is followed by a characteristic hydrophobic stretch of amino acids which probably function as a signal peptide. According to the "-3,-1" rule (von Heijne, G. (1986) Nucl. Ac. Res. 14, 4683-4690), residues 20 and 25 are both likely candidates to constitute the N-terminus of the mature protein. A second hydrophobic stretch is found between amino acids 143 and 166, and is followed by a series of highly charged residues, consistent with the stop-transfer signals found to be associated with many membrane-spanning domains. The predicted intracellular domain of the protein consists of two tandem repeats having 44% sequence identity between each other (residues 259-486 and 552-776). Each of these repeats display significant sequence identity with the intracellular catalytic domains of the previously described transmembrane PTPase CD45 (Ralph, S.J. et al., supra) and LAR (Streuli, M. et al., (1988), supra) (45% and 53% amino acid sequence identity, respectively). In contrast, the EMBL and GENBANK databases contain no significant homology to known sequences of the putative extracellular domain of the encoded protein. Features of the extracellular domain include a uniquely high content of serine and threonine residues (>32%), the absence of cysteine residues, and the presence of 8 potential N-linked glycosylation sites.

It was concluded that the isolated cDNA encoded a new member of the transmembrane PTPase family having a novel type of extracellular domain. In view of its receptor-like structure and the likelihood that additional members of this family can be found based on the present experimental evidence, the name muR-PTPase-α (murine receptor protein tyrosine phosphatase-α) was chosen to designate this protein.

### EXAMPLE II

### Chromosomal Localization of the Mouse R-PTPase-α Gene

STS/A, 020/A, CXS and OXA recombinant inbred (RI) mice, and CXB RI strains N, O, P, Q, and R were a gift from Dr. Jo Hilgers (The Netherlands Cancer Institute). All other inbred mice were purchased from the Jackson Laboratory (Bar Harbor, Maine). Backcross (BC) animals were bred at New York University with inbred progenitors obtained from the Jackson Laboratory. The female parent is named first in all crosses and F1 designations. Spleen genomic DNA from the AKXD, AKXL, BXD, BXH and G, H, I SWXL RI strains, and from CXB, RI strains D, E, G, H, I, J, and K was purchased from the DNA Resource at the Jackson Laboratory. For all other mice, genomic DNA was prepared from crude liver nuclei by a standard sequence of protease digestion, phenol and chloroform extraction, and ethanol precipitation. Mouse genomic DNAs were subjected to Southern blotting analysis by slight modifications of standard procedures, exactly as described previously (Silver, J. (1985) J. Hered. 76, 436-440). A 1.8 kb EcoRI fragment corresponding to the intracellular phosphatase domains of R-PTPase-α, and a 0.7 kb SacII-EcoRI fragment corresponding to its extracellular and transmembrane domains, were cloned into the Bluescript KS vector, yielding plasmids p109 and p923, respectively.

DNA restriction fragment length variants associated with the Il-1a locus (interleukin-1 alpha) were detected by Southern blotting as described previously (D'Eustachio, P. et al., (1987) Immunogenetics 26, 339-343). The significance of deviations from 1:1 segregation for pairs of markers was calculated by the Bayesian method of Silver and Buckler (Silver, J. et al., (1986) Proc. Natl. Acad. Sci. USA 83, 1423-1427); Blank, R.D. et al., (1988) Genetics 120, 1073-1083). Map distances were estimated from recombination fractions measure in RI strain sets according to B.A. Taylor (in: Morse, H.C. III, ed., Origins of Inbred Mice, Academic Press, New York, 1978, pp. 423-438), and their associated 95% binomial confidence limits were calculated according to Silver (1985, supra). Probabilities of alternative orders of trios of markers were calculated according to D. Bishop ((1985) Genet. Epidemiol. 2, 349-361, equation 1). Computations were carried out on a VAX6000-410 computer.

Southern blotting analyses of genomic DNA from inbred strains of mice revealed two useful restriction length variants, one visualized with a probe corresponding to the intracellular domain of muR-PTPase-α (p109) and one visualized with an extracellular and transmembrane domains probe (p923). Together, these variants allowed definition of three allelic forms of muR-PTPase-α among the 10 inbred strains of mice surveyed (Table 1).

**TABLE 1**

| **Restriction Fragment Length Variants Detected by muR-PTPase-α Probes** | | | |
|---|---|---|---|
| Allele | Probe | | Mouse Strains |
| | p109 | p923 | |
| a | 9.4 | 5.9+4.2 | BALB/cJ |
| b | 6.5 | 4.2+1.8 | C57BL/6J, C57L/J, DBA/2J |
| c | 6.5 | 5.9+4.2 | C3H/HeJ, 020/A, AKR/J, |
| | | | SWR/J, SJL/J, STS/A |
| Liver genomic DNA digested with TagI restriction endonuclease was analyzed by Southern blotting. Fragment sizes in kilobases are shown. | | | |

Inheritance of these alleles in RI mice was scored. Comparison of the strain distribution patterns observed for muR-PTPase-α (Table 2) with those previously observed for other markers of known chromosomal location in these mice indicated close linkage between the muR-PTPase-α and Il-1a (Interleukin-1) loci on chromosome 2 (3 RI strains among 89 examined). This degree of concordance has a probability of less than 0.00001 of occurring as a chance event were the loci unlinked. The observed fraction of recombinant strains indicates a map distance of 0.9 cM between the loci (95% confidence limits 0.2 - 0.6 cM).

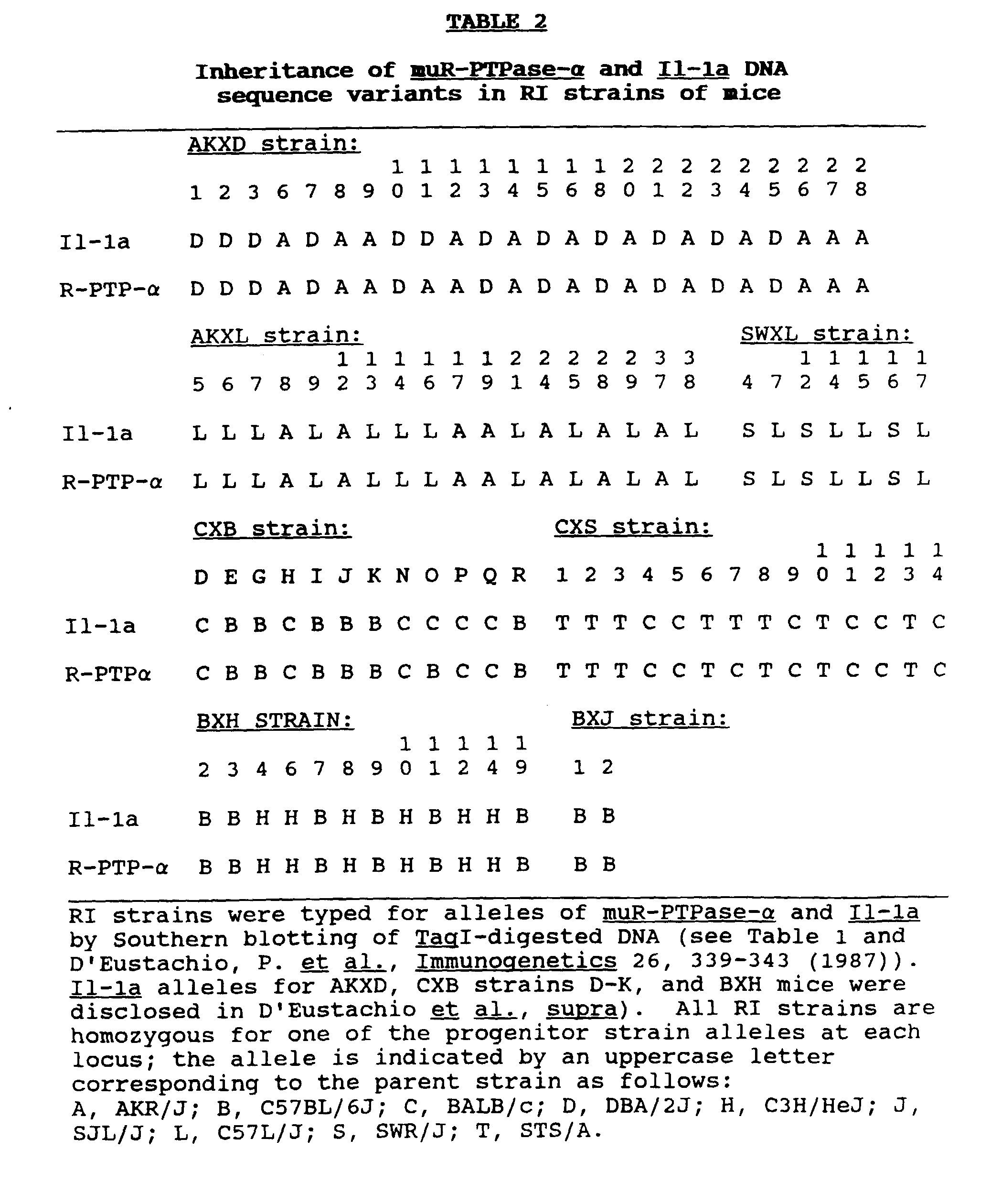

Following the inheritance of muR-PTPase-α, Il-1a and a (nonagouti) among progeny of reciprocal backcross between the C57BL/6J and SWR/J strains confirmed the linkage of muR-PTPase-α and Il-1a, and suggested an order for the two genes (Table 3). Of 150 progeny, 14 were recombinant between muR-PTPase-α and a, and one was recombinant between muR-PTPase-α and Il-1a. If the locus order were: centromere-Il-1α-muR-PTPase-α-a, these results would require the occurrence of no double crossovers; alternative orders require one or 14 such events, and, evaluated according to the method of Bishop (supra), are at least 9.5-fold less likely. The distance between Il-1a and muR-PTPase-α , 0.6 cM (95% confidence limits: 0.1-2.4 cM), agrees within sampling fluctuation with the distance estimated from the RI strain data. Comparison of these results with results recently obtained for Bmp-2a (Bone morphogenic protein 2a, Dickinson, M.E. et al., (1990) Genomics 6, 505-520) suggests that the two genes may be closely linked, although there is no obvious structural homology between them.

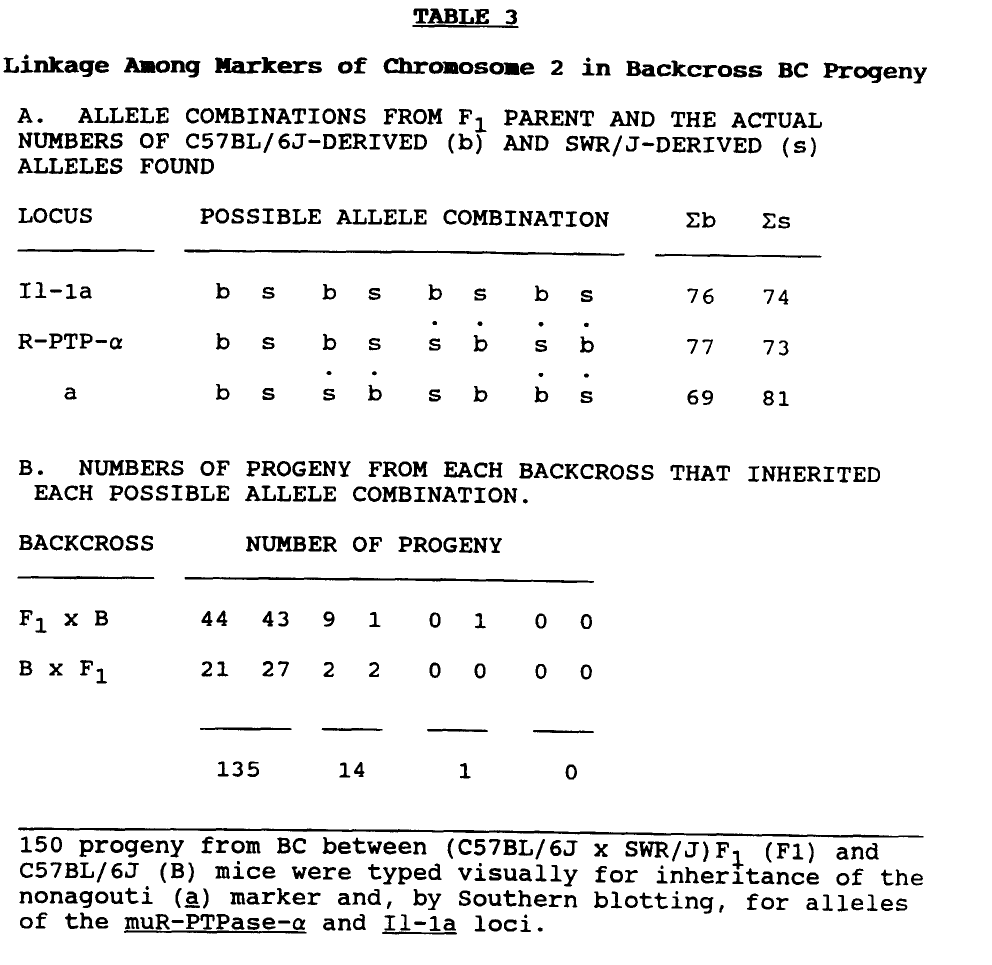

### EXAMPLE III

### Expression of the Murine R-PTPase-α RNA

### 1. Northern Analysis.

Poly A⁺ RNA was prepared from adult mouse tissues and cell lines by oligo(dT) selection as described (Vennström, B. et al., (1982) Cell 28, 135-143), fractionated (5µg per lane) on a formaldehyde-containing gel and transferred to nitrocellulose (Hybond C, Amersham) using standard procedures. A ³²P-labelled probe was prepared by primer extension on a single-stranded template consisting of the entire lambda-109 cDNA cloned into the EcoRI site of the Bluescript vector in the antisense orientation, using the Klenow fragment of DNA polymerase for elongation from an annealed T7 primer, in the presence of ³²P- dATP. Hybridization was performed at 42°C in 50% formamide, 5xSSC, 25 mM KPO₄, 5X Denhardt's, 10 µg/ml salmon sperm DNA, and 10% sulfate. Washing was done at 48°C in 0.1X SSC, 0.1% SDS. Higher stringency washes (58°C) of the filter did not noticeably affect the hybridization pattern.

### 2. Expression of the Murine R-PTPase-α Drotein.

The entire cDNA insert from phage lambda-109 was released as one fragment from the phage using partial EcoRI digestion, and cloned into the Bluescript KS vector. A cDNA fragment lacking most of the untranslated leader sequence (starting from the Sac II site at position 226; see Figure 1b) was subcloned into the SV40 promoter driven pLSV-vector (Livneh, E., et al., (1986) J. Biol. Chem. 261, 12490-12497), and the resulting plasmid DNA (pLSV-PTP-α) was transfected into COS cells using the DEAE-dextran method (Lopata, M.A. et al., (1984) Nucl. Ac. Res. 12, 5707-5717). The expression vector pLSV C encoding the N-terminally truncated muR-PTPase-α protein was used as a control in the immunoprecipitation experiment.

### 3. Results

Poly A⁺ RNA from various mouse tissues was prepared to study the expression of the muR-PTPase-α gene. Northern analysis (Figure 2) revealed a wide pattern of expression. A 3.0 kB mRNA was present in all tissues examined, except spleen, with brain and kidney showing the highest levels of expression. An mRNA of similar size could also be observed in the NIH-3T3 mouse fibroblast line, 2.2, and the prepro-B lymphoid cell line, BAF (Figure 2). Shorter exposure of the Northern blot clearly showed that in addition a second mRNA species of very similar size (3.2 kb) is present in several tissues (e.g. brain) in lower amounts. The data also suggest that, although a poly A tail and a polyadenylation signal at the 3' end of the cDNA sequence were not observed, the isolated cDNA clone (2872 nucleotides) closely matches the full length of the mRNA.

### EXAMPLE IV

### Transient Expression of the Murine R-PTPase-α Protein

### 1. Antibody Preparation and Immunoprecipitation.

Rabbits were injected with a synthetic peptide corresponding to the predicted C-terminus of the muR-PTPase-a protein (residues 777-794) coupled to BSA using EDCI (1-ethyl-3- (dimethylaminopropyl)carbodiimide) as a coupling reagent. Antigen was injected intradermally and subcutaneously in an emulsion of 1 mg peptide and complete Freund's adjuvant. Three booster injections were given at 2-3 week intervals with 0.5 mg peptide and incomplete adjuvant. An antiserum obtained using this method was designated "2A." Metabolic [³⁵S]-methionine labelling, cell extract preparation (60 hours after transfection) and indirect immunoprecipitation using protein-A-Sepharose were performed using standard procedures (Yarden, Y. et al., (1987) EMBO J. 6, 3341- 3351).

### 2. Results

In order to determine the size of the mature protein, we cloned the muR-PTPase-α cDNA with the exception of most of the untranslated leader into the pLSV vector (Livneh, E., et al., (1986) J. Biol. Chem. 261, 12490-12497) under the control of the SV40 promoter, yielding the expression vector pLSV-PTP-α. The vector was transfected into COS cells, and 60 hours later [³⁵S]-methionine labelled total cell extracts were prepared for immunoprecipitation, using antiserum 2A.

As seen in Figure 3, the antiserum recognized several bands, one of which, a diffuse band of 130 kDa (arrow), was only present in immunoprecipitates from transfected cells (lane 5), but not from mock-transfected cells (lane 3) (transfected with pLSV without the muR-PTPase-α cDNA). Precipitation could be competed out by the peptide used for immunization (lane 6).

The difference between the predicted (88 kDa) and observed (130 kDa) molecular weights for the muR-PTPase-α protein is ascribed to its extensive glycosylation.

As an additional control for the specificity of the antiserum, we also transfected COS cells with a N-truncated version of the muR-PTPase-α cDNA (starting at amino acid 214, and thus lacking the transmembrane and extracellular domains) in the same vector. A new and abundant protein with an apparent molecular weight of 55 kDa appeared in immunoprecipitates from cells transfected with this vector, which was again competed out by the antigenic peptide (lanes 7 and 8). The higher abundance of the truncated protein as compared to the mature muR-PTPase-α protein was a consistent observation over several independent transfection experiments.

### General Discussion for Examples I-IV

The Examples presented above describe the identification of a novel receptor-like PTPase, R-PTPase-α, having a broad pattern of expression. R-PTPases are therefore expected to have widespread functions beyond the regulation of lymphoid cell activity, as was previously thought based on study of CD45.

Studies using monoclonal antibodies directed against the extracellular domain of CD45 proteins showed that cross-linking of R-PTPases can have profound effects on various cellular activities, although a direct effect on PTPase enzymatic activity remains to be shown. However, since ligand-induced receptor clustering is a central event in transmembrane signalling by receptor tyrosine kinases (Ullrich, A. et al., supra), it is proposed by the inventors that putative extracellular ligands for R-PTPases have the capacity to regulate the activity of R-PTPases in vivo.

In a manner analogous to that proposed for receptor tyrosine kinases (PTKs), R-PTPases are proposed to have arisen through several gene fusion events between an ancestral PTPase domain, and domains capable of binding extracellular ligands (Ullrich, A. et al., Hanks, S.K. et al., supra).

The variety of extracellular domains potentially joined to PTPase domains to form receptor-like proteins are expected to reflect the range of possible ligands able to act by similar mechanisms. The availability of cloned R-PTPases, such as those disclosed herein, will be valuable in determining their substrate specificity and in understanding their function and manipulating their activity.

R-PTPases might have a broad specificity directed towards major tyrosine kinase substrates, with their different extracellular domains mainly allowing for different regulatory mechanisms responsive to different signals in the extracellular environment. Based on this view, they are expected to modulate the responsiveness of a cell to those polypeptide growth factors which act through receptor protein tyrosine kinases. As with PTK's, ligand binding would lead to an activation of enzymatic activity. Viewed in this light, R-PTPase-α and molecules like it, would be negative growth regulators and can be considered potential recessive oncogenes.

For instance, deletion of portions of murine chromosome 2, to which R-PTPase-α maps, appears to be an early event in the development of radiation-induced myeloid leukemia in SJL/J mice (Tracktenbrot, L. et al., (1988) Leukemia 2, 545-550), consistent with the recessive oncogene notion. Furthermore, rearrangements involving human chromosome 20 (to which the human R-PTPase-α gene maps) have been linked to human lymphoid leukemia (Mitelman, F. (ed.) Catalog of Chromosome Aberrations in Human Cancer, A. Liss, New York).

Alternatively, R-PTPase-α may act in a manner analogous to that proposed for the interaction between CD45 and c-lck (Oostergaard, H.L. et al., (1989) Proc. Natl. Acad. Sci. USA 86, 8959-8963; Mustelin, T. et al., (1989) Proc. Natl. Acad. Sci. USA 86, 6302-6306). According to this view, R-PTPase-α would dephosphorylate negative regulatory sites in membrane-associated PTKs which are not receptors, and which are more widely expressed than lck (such as, for example, the tyr⁵²⁷ site in pp60^{c-src}). Acting in this manner, R-PTPase-α would be implicated in positive growth control and differentiation.

Although the inventors do not intend to be bound by any particular theory, the high interspecies conservation of the catalytic domains of the various receptor-PTPases indicate an important role for these receptors in cell growth control.

### EXAMPLE V

Isolation and Characterization of Human R-PTPase cDNA (Kaplan, R. et al., Proc. Natl. Acad. Sci. USA 87:7000-7004 (1990))

### A. Materials

Restriction endonucleases and modifying enzymes were purchases from Boehringer-Mannheim or New England Biolabs. Taq DNA polymerase was from Perkin-Elmer/Cetus. The lambda gtll forward and reverse primers (24-mers) used in the polymerase chain reactions as well as all sequencing primers, were synthesized on an automated DNA synthesizer (Applied Biosystems, model 380A) using either methoxy or β-cyanoethyl phosphoramidites (House, C., et al., J. Biol. Chem., 262:772-777 (1987)). The lambda gtll human brainstem cDNA library was obtained form the American Type Culture Collection (no. 37432). The LCA (CD45) clone used as a probe for screening the library was received from E.H. Fischer (University of Washington, Seattle). All sequencing reactions were performed using the Sequenase kit (United States Biochemical).

### B. Methods

Approximately 300,000 plaques from a lambda gtll cDNA library of 1-day-old human infant brainstem were screened on duplicate nitrocellulose filters under conditions of reduced stringency with a nick-translated LCA probe that spanned both conserved phosphatase domains (Charbonneau, H. et al., 1989, supra).

Hybridization was carried out at 55°C overnight in a solution of 5x SSPE (SSPE is 10 mM NaH₂PO₄, pH 7.4/0.18 M NaCl/l mM EDTA) containing 0.25% nonfat dry milk, 0.1% SDS, and ³²P-labeled LCA probe at 10⁶ cpm/ml. The filters were washed three times for 20 min at 55°C in 2 x SSPE/0.2% SDS and then processed for autoradiography. This screen yielded 79 duplicate positives; 12 of these, showing varying degrees of hybridization to the LCA probe, were plaque-purified by repetition screening with the same probe. The polymerase chain reaction (Saiki, R.K., et al., Science, 230:1350-1354 (1985)) was then used to determine the sizes of the cDNA inserts. The DNA templates consisted of portions of the eluates from each pure plaque, heated at 75°C for 15 min. to release the DNA. The templates were primed with the lambda gtll forward and reverse primers. The reaction mixtures (0.1 ml) were prepared as described (Dionne, C.A. et al., Biotechniques 8:190-194 (1990)). Amplification was achieved by performing 30 cycles, each including 1.5 min of denaturation at 94°C, 2 min of annealing at 65°C, and 4 min of extension at 72°C, in an automated Perkin-Elmer/Cetus DNA thermal cycler. A portion of each sample (15 µl) was analyzed by electrophoresis through a 1% agarose gel containing ethidium bromide at 1 µg/ml (Sambrook et al., supra). DNA was prepared from the 4 largest clones by using LambdaSorb (Promega) and then digested with EcoRI. The fragments were subcloned separately into the EcoRI site of M13mp18 for sequencing. Nucleotide sequences were determined by the dideoxynucleotide chain-termination method (Sanger, F., et al., Proc. Natl. Acad. Sci. USA, 74:5463-5467 (1977)) using modified T7 polymerase (Tabor, S. et al., Proc. Natl. Acad. Sci. USA 84:4767- 4771 (1987)).

All computer analyses of sequence data were performed on a Micro VAX II using programs written by IntelliGenetics. DNA sequences were analyzed and assembled using the GEL program. Hydrophobic analyses of proteins were based on the algorithm of Kyte and Doolittle (Kyte, J. et al., J. Mol. Biol. 157:105-132 (1982)), as implemented in the PEP program. Protein sequence alignments were done using the GENALIGN program (Sobel, E. et al., Nucleic Acids Res. 14:363-374 (1985); Karlin, S. et al., Mol. Biol. Evol. 1:357-370 (1984); Needleman, S.B. et al., J. Mol. Biol. 48:443-453 (1970)). Initial alignments were done using the Jimenez-Montano protein alphabet (Jimenez-Montano, M. et al., Proc. 7th Int'l. Biophysics Congress, 1981, Mexico City).

### C. Results

In an effort to identify new members of the PTPase family, 300,000 plaques from a human infant brainstem cDNA library in lambda gt11 were screened under nonstringent conditions using a nick-translated LCA probe that spanned both conserved phosphatase domains. Four of the initial 79 duplicate positives were sequenced in the entirety. Two clones, 31-4 and 27-1, contained overlapping portions of the entire coding region of a human R-PTPase (huR-PTPase) that was designated α (Figure 4B). The combined lengths of clones 31-4 and 27-1 equaled 3615 bp (Figure 4A), encoding a protein of 802 amino acids (Figure 4C) and containing an additional 695 bp and 510 bp, respectively, of 5' and 3' untranslated region. Two of the four clones contained portions of genes coding for two additional R-PTPases which have been designated β and gamma (Figure 5). Like R-PTPase-α, these two proteins contain typical hydrophobic transmembrane regions and distinct extracellular domains, indicating that they also represent separate R-PTPases.

The murine homologue of this gene was cloned and sequenced at the same time as the human R-PTPase-α (see Examples I-IV, above). A comparison of the mouse and human protein sequences is shown in Figure 4C. With the exception of the extracellular domain, where some variability exists, only 5 residues are found to differ between the two proteins. An examination of the structure of human R-PTPase-α reveals the following features: a relatively short extracellular domain consisting of 150 residues that includes a hydrophobic signal peptide containing the only cysteine in this region. There are eight potential N-glycosylation sites, as well as a number of potential O-glycosylation sites (since this domain is rich in serine and threonine). The extracellular domains of R-PTPase-α, LCA, and LAR appear to be structurally unrelated. There is a hydrophobic transmembrane region anchored on both sides by charged residues. This is followed by the two tandemly repeated conserved phosphatase domains of about 235 residues each, which are separated by 57 amino acids, typical of R-PTPases such as LCA, LAR and the two Drosophila PTPases, DLAR and DPTP.

Figures 5A and 5B show the alignments of the amino acids within the first and second conserved phosphatase domains, respectively, of LCA and R-PTPases α, β, and gamma. It is readily apparent that among the four R-PTPases, β and gamma share the greatest sequence similarity. It was reported (Hunter, T. et al. supra) that among the sequences of the conserved phosphatase domains of PTPase 1B, LCA, LAR, DLAR and DPTP there are 29 invariant residues. While many of these residues are also present in both phosphatase domains of R-PTPase-α, β, and gamma, it is interesting that the second conserved phosphatase domains of both β and gamma lack a number of these amino acids, including the two cysteines at positions 104 and 201 in phosphatase domain 2 of LCA (see Figure 5B).

### D. Discussion

The sequences of the conserved phosphatase domains of the three human R-PTPases identified here (α, β, and gamma) have been compared with one another as well as with those of LCA, LAR, and two soluble PTPases, placental phosphatase 1B and T-cell PTPase (Table 4). The two soluble enzymes have a sequence identity of 70%; however, when each is compared with the R-PTPases (Phosphatase domains PD1 or PD2), this number drops to 29-42%. In all cases, the soluble PTPases showed a greater identity with PD1 than with PD2 of the R-PTPases. R-PTPase-α appears to be most related to LAR, since their PD1 sequences are 56% identical and their PD2 sequences are 52% identical. The conserved domains of R-PTPases β and gamma are most related to each other, even more so than are the two soluble PTPases, β and gamma being 75% identical in both PD1 and PD2. It is interesting that, in general, the sequence relationship between PD1 and PD2 within any R-PTPase appears to be no closer than that seen between different members of the family, i.e., the identities between PD1 and PD2 range from a high of 47% for LAR to a low of 29% for R-PTPase gamma.

While the cytoplasmic domains of R-PTPase-α, β, and gamma are highly conserved, the extracellular domains of these receptors are unrelated to one another as well as to those of LAR and LCA. This suggests that each of these receptors has its own distinct ligand. It is likely that the binding of such ligands to the R-PTPases plays a crucial role, together with growth factor receptors exhibiting PTKase activity, in the regulation of the level of tyrosine phosphorylation of targets proteins involved in signal transduction. The diversity of the R-PTPases described herein reveals the existence of a multigene family. Greater understanding of structure-function relationships among these membrane receptors will provide important insights into the mechanisms involved in cell growth, differentiation, and oncogenesis.

**Table 4**

| Identities Between Conserved Phosphatase Domains (Percent) | | | | | | | | | | | | |
|---|---|---|---|---|---|---|---|---|---|---|---|---|
| | PTPase | T-cell | LCA | | LAR | | RPTPase-α | | RPTPase-β | | RPTPase-gamma | |
| | 1B | PTPase | PD1 | PD1 | PD1 | PD2 | PD1 | PD2 | PD1 | PD2 | PD1 | PD2 |
| | | | | | | | | | | | | |
| PTPase 1B | 100 | .. | .. | .. | .. | .. | .. | .. | .. | .. | .. | .. |
| T-cell PTPase | 70 | 100 | .. | .. | .. | .. | .. | .. | .. | .. | .. | .. |
| LCA PD1 | 37 | 36 | 100 | .. | .. | .. | .. | .. | .. | .. | .. | .. |
| LCA PD2 | 30 | 26 | 31 | 100 | .. | .. | .. | .. | .. | .. | .. | .. |
| LAR PD1 | 39 | 42 | 50 | 28 | 100 | .. | .. | .. | .. | .. | .. | .. |
| LAR PD2 | 29 | 33 | 42 | 34 | 45 | 100 | .. | .. | .. | .. | .. | .. |
| R-PTPase-α PD1 | 36 | 38 | 50 | 32 | 56 | 45 | 100 | .. | .. | .. | .. | .. |
| R-PTPase-α PD2 | 33 | 34 | 40 | 32 | 41 | 52 | 43 | 100 | .. | .. | .. | .. |
| R-pTPase-β PD1 | 35 | 39 | 41 | 31 | 33 | 41 | 47 | 33 | 100 | .. | .. | .. |
| R-PTPase-β PD2 | 29 | 30 | 31 | 30 | 31 | 34 | 31 | 37 | 30 | 100 | .. | .. |
| R-PTPase-gamma PD1 | 35 | 34 | 32 | 29 | 39 | 36 | 34 | 32 | 75 | 27 | 100 | .. |
| R-PTPase-gamma PD2 | 29 | 29 | 30 | 28 | 32 | 36 | 31 | 34 | 33 | 75 | 29 | 100 |
| Alignments of the conserved phosphatase domains were carried out as described above. The regions compared are designated in Figure 4C and Figure 5. PD=phosphatase domain. | | | | | | | | | | | | |

### EXAMPLE VI

### Expression of Human R-PTPase-α by Northern Blot Analysis

Samples containing either 20 µg of total RNA or 2 µg of poly(A)⁺ RNA were resolved in a formaldehyde/agarose gel and transferred to nitrocellulose. R-PTPase-α and β-actin probes were labeled by random priming (Sambrook et al., supra). Hybridizations and washes were carried out at 65°C as described (Church, G., et al., Proc. Natl. Acad. Sci. USA, 81:1991- 1995 (1984)). Blots hybridized with the R-PTPase-α probe were exposed to XAR-2-x-ray film (Kodak) with an intensifying screen for 72 hr at -80°C. Results were obtained from the actin-probe blots after 15 hr under the same conditions.

R-PTPase-α expression was examined in various cell lines and tissues (Figure 6). The results indicate the presence of two major RNA transcripts of approximately 4.3 and 6.3 kb, respectively. The larger of the two species appears to be more prevalent in fetal tissues and in particularly prominent in the poly(A)⁺ fetal liver sample, where there is also the highest relative amount of the 4.3-kb transcript. It is possible that the different expression of the two transcripts is developmentally regulated and/or a result of alternative splicing mechanisms, a feature seen with LCA (Ralph, S.J. supra). The adult brain shows relatively less expression of R-PTPase-α. The results suggest that R-PTPase-α is expressed to some degree throughout many tissues. Murine R-PTPase-α was also shown to be expressed in many tissues and cell lines and most abundantly in brain and kidney (Sap, J., et al., Proc. Natl. Acad. Sci. USA, 87:6112- 6116, (1990) see also Examples III and IV, above).

### EXAMPLE VII

### Chromosome Localization of the Human R-PTPase-α Gene

Isolation, propagation, and characterization of parental and somatic cell hybrids using in this study have been described (Durst, M. et al., Proc Natl. Acad. Sci. USA 84:1070- 1074 (1987); Ku, D-H. et al., Somatic Cell Mol. Genet. 15:297-307 (1989); Juan, C-C. et al., Proc. Natl. Acad. Sci. USA 85:8910-8913 (1988)). Presence of specific human chromosomes or regions of chromosomes has been confirmed by DNA hybridization using probes for genes assigned to specific chromosome regions. Hybrid DNAs were digested with an excess of restriction endonuclease HindIII or EcoRI, sized by electrophoresis in 0.8% agarose gels, transferred to nylon filters, and hybridized as described (Durst et al., supra). The R-PTPase-α probe consisted of the 3'-most 0.8 kilobases (kb) of clone 31-4 (see Figure 4B).

DNAs from 17 rodent-human somatic cell hybrids carrying overlapping subsets of human chromosome regions representing the entire human genome were tested for presence of the human R-PTPase-α locus by Southern blot analysis. The results (Figure 7) show that presence of the human R-PTPase-α locus in hybrid cells correlates only with presence of a partial human chromosome 20. The data also allow a regional localization for the R-PTPase-α locus, since hybrids PB5-1 and AB3 are each missing a part of the long arm of chromosome 20 and yet retain the R-PTPase-α locus. Thus, the human R-PTPase-α gene maps to 20pter-20q12.

It has been observed (Lalley, P.A. et al., Cytogenet. Cell Genet. 51:503- 532 (1989)) that the murine homologues of all human genes which have been mapped to human chromosome 20 map to mouse chromosome 2. This appears to be true for R-PTPase-α as well (see Example II, above). The long arm of human chromosome 20 is involved in translocation and deletions in myeloid disorders and neoplasms (Trent, J.M., et al., Cytogenet. Cell Genet., 51:533-562, (1989)). The human R-PTPase-α locus may be specifically involved in deletion on 20q; in this case, it would strengthen the possibility of it being a tumor-suppressor gene or anti-oncogene. Similarly in mice, in the SJL/J strain, deletion of chromosome 2 appears to be involved in the development of radiation-induced myeloid leukemia (Trakhtenbrot, L., et al., Leukemia, 2:545-550, (1988)).

The references cited above are all incorporated by reference herein, whether specifically incorporated or not.

Having now fully described this invention, it will be appreciated by those skilled in the art that the same can be performed within a wide range of equivalent parameters, concentrations, and conditions without departing from the spirit and scope of the invention and without undue experimentation.

While this invention has been described in connection with specific embodiments thereof, it will be understood that it is capable of further modifications. This application is intended to cover any variations, uses, or adaptations of the inventions following, in general, the principles of the invention and including such departures from the present disclosure as come within known or customary practice within the art to which the invention pertains and as may be applied to the essential features hereinbefore set forth as follows in the scope of the appended claims.

## Claims

1. An isolated human receptor-type protein tyrosine phosphatase α protein or glycoprotein as depicted in line 1 of Figure 4.

2. An isolated nucleic acid molecule encoding a human receptor-type protein tyrosine phosphatase α protein or glycoprotein as shown in line 1 of Figure 4.

3. A nucleic acid molecule according to claim 2 which is a cDNA molecule.

4. A nucleic acid molecule according to claim 2 which is a genomic DNA molecule.

5. A nucleic acid molecule encoding the protein according to claim 2.

6. A recombinant vector complaining the nucleic acid molecule of claim 2 or 5.

7. An expression vector containing the nucleic acid molecule of claim 2 or 5 operatively linked with an element that controls expression of the nucleic acid molecule in a host cell.

8. A genetically engineered host cell containing the nucleic acid molecule of claim 2 or 5.

9. A genetically engineered host cell containing the vector of claim 6 or 7.

10. The genetically engineered host cell of claim 6 or 7 which is eukaryotic.

11. The genetically engineered host cell of claim 6 or 7 whir is prokaryotic.

12. A process for preparing a human receptor-type protein tyrosine phosphatase α protein or glycoprotein, according to claim 1, said process comprising :
(a) culturing a host capable of expressing said protein, glycoprotein under culturing conditions,
(b) expressing said protein or glycoprotein; and
(c) recovering said protein or glycoprotein from said culture.

13. A method for detecting the presence of a nucleic acid molecule according to claim 2, or a mutant allele thereof, in a subject, comprising :
(a) containing a cell or an extract thereof from said subject with a nucleic acid probe encoding at least a portion of normal human receptor-type protein tyrosine phosphatase α or said mutant allele thereof under hybridizing conditions; and
(b) measuring the hybridization of said probe to the nucleic acid of said cell,
thereby detesting the presence of said nucleic acid molecule.

14. The method of claim 13, additionally comprising before step (a) :
(c) selectively amplifying the amount of DNA of said cell encoding said receptor-type protein tyrosine phosphatase.

15. A pharmaceutical composition for treating or preventing a disease associated with an abnormal receptor-type protein tyrosine phosphatase-α, said composition comprising a pharmaceutically acceptable carrier and a therapeutically effective amount of the protein or glycoprotein or polypeptide of claim 1.

16. A method for isolating from a mixture of chemical or biological preparation a compound capable of binding receptor-type protein tyrosine phosphatase-α comprising :
(a) contacting the mixture with the protein or glycoprotein or polypeptide of claim 1 attached to a solid phase matrix under conditions and for a time sufficient for binding to occur ;
(b) removing unbound components; and
(c) eluting any compound bound to the protein or glycoprotein or polypeptide from the solid phase and collecting the eluted compound.

17. A method for identifying a compound capable of stimulating or inhibiting the enzymatic activity of receptor-type protein tyrosine phosphatase-α, comprising :
(a) incubating said compound with the protein or glycoprotein or polypeptide of claim 1 in pure form, in a membrane preparation, or in a whole cell;
(b) measuring the enzymatic activity of the protein or glycoprotein or polypeptide; and
(c) comparing the enzymatic activity to that of the protein or glycoprotein or polypeptide incubated without said compound, thereby determining whether said compound stimulates or inhibits receptor-type protein tyrosine phosphatase-α enzymatic activity.

## Patentansprüche

1. Ein isoliertes humanes Protein-Tyrosin-Phosphatase-α-Protein oder -Glykoprotein vom Rezeptor-Typ, wie es in Zeile 1 von Figur 4 gezeigt ist.

2. Ein isoliertes Nukleinsäuremolekül, das für ein humanes Protein-Tyrosin-Phosphatase-α-Protein oder -Glykoprotein vom Rezeptor-Typ kodiert, wie es in Zeile 1 von Figur 1 gezeigt ist.

3. Ein Nukleinsäuremolekül nach Anspruch 2, das ein cDNA-Molekül ist.

4. Ein Nukleinsäuremolekül nach Anspruch 2, das ein Genom-DNA-Molekül ist.

5. Ein Nukleinsäuremolekül, das für das Protein nach Anspruch 2 kodiert.

6. Rekombinanter Vektor, der das Nukleinsäuremolekül nach Anspruch 2 oder 5 enthält.

7. Expressionsvektor, der das Nukleinsäuremolekül nach Anspruch 2 oder Anspruch 5, das operativ mit einem Element verknüpft ist, das die Expression des Nukleinsäuremoleküls in einer Wirtszelle kontrolliert, enthält.

8. Eine gentechnisch veränderte Wirtszelle, die das Nukleinsäuremolekül nach Anspruch 2 oder 5 enthält.

9. Gentechnisch veränderte Wirtszelle, die den Vektor nach Anspruch 6 oder 7 enthält.

10. Gentechnologisch veränderte Wirtszelle nach Anspruch 6 oder 7, die eukaryotisch ist.

11. Gentechnisch veränderte Wirtszelle nach Anspruch 6 oder 7, die prokaryotisch ist.

12. Verfahren zur Herstellung eines humanen Protein-Tyrosin-Phosphatase-α-Proteins oder -Glykoproteins vom Rezeptor-Typ nach Anspruch 1, wobei das Verfahren umfaßt:
(a) Kultivieren eines Wirts, der in der Lage ist, das genannte Protein, Glykoprotein unter Kultivierungsbedingungen zu exprimieren,
(b) Exprimieren des genannten Proteins oder Glykoproteins; und
(c) Gewinnen des genannten Proteins oder Glykoproteins aus der genannten Kultur.

13. Verfahren zum Nachweis der Anwesenheit eines Nukleinsäuremoleküls nach Anspruch 2 oder eines Mutantenallels davon in einem Lebewesen, das umfaßt:
(a) Inkontaktbringen einer Zelle aus dem geannten Lebewesen oder eines Extrakts daraus mit einer Nukleinsäuresonde, die für wenigstens einen Teil einer normalen humanen Protein-Tyrosin-Phosphanase-α vom Rezeptor-Typ oder des genannten Mutantenallels davon kodiert, unter Hybridisierungsbedingungen; und
(b) Messen der Hybridisierung der genannten Sonde an die Nukleinsäure der genannten Zelle,
wodurch die Anwesenheit des genannten Nukleinsäuremoleküls nachgewiesen wird.

14. Verfahren nach Anspruch 13, das zusätzlich vor Stufe (a) umfaßt:
(c) die selektive Vervielfältigung der DNA-Menge der genannten Zelle, die für die genannte Protein-Tyrosinphosphatase vom Rezeptor-Typ kodiert.

15. Pharmazeutische Zusammensetzung zur Behandlung oder Prävention einer Erkrankung, die mit einer anormalen Protein-Tyrosin-Phosphatase-α vom Rezeptor-Typ verbunden ist, wobei die genannte Zusammensetzung einen pharmazeutisch zulässigen Träger und eine therapeutisch wirksame Menge des Proteins oder Glykoproteins oder Polypeptids nach Anspruch 1 enthält.

16. Verfahren zur Isolierung aus einer chemischen oder biologischen Präparation einer Verbindung, die in der Lage ist, die Protein-Tyrosin-Phosphatase-α vom Rezeptor-Typ zu binden, das umfaßt:
(a) Inberührungbringen der Mischung mit dem Protein oder Glykoprotein oder Polypeptid nach Anspruch 1, das an eine Festphasenmatrix gebunden ist, unter solchen Bedingungen und für eine solche Zeit, die ausreichen, daß es zu einer Bindung kommt;
(b) Entfernen nicht gebundener Komponenten; und
(c) Eluieren der gesamten Verbindung, die an das Protein cder Glykoprotein oder Polypeptid gebunden ist, von der Festphase und Sammeln der eluierten Verbindung.

17. Verfahren zur Identifizierung einer Verbindung, die in der Lage ist, die enzymatische Aktivität von Protein-Tyrosin-Phosphatase-α vom Rezeptor-Typ zu stimulieren oder inhibieren, das umfaßt:
(a) Inkubieren der genannten Verbindung mit dem Protein oder Glykprotein oder Polypeptid nach Anspruch 1 in reiner Form, in einer Membranpräparation oder in einer ganzen Zelle;
(b) Messen der Enzymaktivität des Proteins oder Glykoproteins oder Polypeptids; und
(c) Vergleichen der Enzymaktivität mit der des Proteins oder Glykoproteins oder Polypeptids, mit dem die genannte Verbindung inkubiert wurde, um dadurch festzustellen, ob die genannte Verbindung die enzymatische Aktivität der Protein-Tyrosin-Phosphatase-α vom Rezeptor-Typ stimuliert oder inhibiert.

## Revendications

1. Glycoprotéine ou protéine de protéine de tyrosine phosphatase α du type récepteur humaine isolée telle que décrite à la ligne 1 de la figure 4.

2. Molécule d'acides nucléiques isolée encodant une protéine ou glycoprotéine de protéine de tyrosine phosphatase α du type récepteur telle que montrée à la ligne 1 de la figure 4.

3. Molécule d'acides nucléiques selon la revendication 2 qui est une molécule d'ADNc.

4. Molécule d'acides nucléiques selon la revendication 2 qui est une molécule d'ADN génomique.

5. Molécule d'acides nucléiques encodant la protéine selon la revendication 2 .

6. Vecteur recombinant contenant la molécule d'acides nucléiques selon la revendication 2 ou 5.

7. Vecteur d'expression contenant la molécule d'acides nucléiques de la revendication 2 ou 5 pontée opérationellement à un élément qui contrôle l'expression de la molécule d'acides nucléiques dans une cellule hôte.

8. Cellule hôte manipulée génétiquement contenant la molécule d'acides nucléiques de la revendication 2 ou 5.

9. Cellule hôte manipulée génétiquement contenant le vecteur de la revendication 6 ou 7.

10. Cellule hôte manipulée génétiquement de la revendication 6 ou 7 qui est eucariotique.

11. Cellule hôte manipulée génétiquement de la revendication 6 ou 7 qui est procariotique.

12. Procédé pour préparer une protéine ou glycoprotéine de protéine de tyrosine phosphatase α contenant :
(a) la culture d'un hôte capable d'exprimer ladite protéine ou glycoprotéine dans des conditions de ladite culture.
(b) l'expression de ladite protéine ou glycoprotéine ; et
(c) la récupération de ladite protéine ou glycoprotéine de ladite culture.

13. Méthode pour détecter la présence d'une molécule d'acides nucléiques selon la revendication 2, ou un allèle mutant de celle-ci dans un sujet comprenant :
(a) la mise en contact d'une cellule ou d'un extrait de celle-ci provenant dudit sujet avec une sonde d'acides nucléiques encodant au moins une portion d'une protéine de tyrosine de phosphatase α du type récepteur humaine normale ou ledit allèle mutant de celle-ci dans des conditions d'hybridation ;
(b) la mesure de l'hybridation de ladite sonde à l'acide nucléique de ladite cellule.
pour ainsi détecter la présence de ladite molécule d'acides nucléiques.

14. Méthode selon la revendication 13, comprenant addtionellement avant l'étape (a) :
(c) l'amplification sélectivement de la quantité d'ADN de ladite cellule encodant ladite protéine de tyrosine phosphatase du type récepteur

15. Composition pharmaceutique pour traiter ou prévenir une maladie associée à une protéine de tyrosine phosphatase α du type récepteur anormale, ladite compostion comprenant un porteur pharmaceutiquement acceptable et une quantité thérapeutiquement efficace de la protéine ou glycoprotéine ou du polypetide de la revendication 1.

16. Méthode pour isoler d'un mélange d'une préparation chimique ou biologique un composé capable de liaison à une protéine de tyrosine phosphatase α du type récepteur comprenant :
(a) la mise en contact du mélange avec la protéine ou la glycoprotéine ou le polypetide de la revendication 1 attaché à une matrice de phase solide dans des conditions et pendant un temps suffisants pour que la liaison se produise ;
(b) l'élimination des composants non liés :
(c) l'alution dudit composé lié à la protéine ou à la glycoprotéine ou au polypetide de la phase solide et de la collecte du composant élué.

17. Méthode pour identifier un composé capable de stimuler ou d'inhiber l'activité enzymatique de la protéine de tyrosine phosphatase α du type récepteur comprenant :
(a) l'incubation dudit composé avec la protéine ou la glycoprotéine ou le polypeptide de la revendication 1 sous forme pure dans une préparation de membrane, ou dans la cellule entière :
(b) la mesure de l'activité enzymatique de la protéine ou de la glycoprotéine ou du polypetide ; et
(c) la comparaison de l'activité enzymatique à celle de la protéine ou de la glycoprotéine ou du polypetide incubé sans ledit composé pour ainsi déterminer si ledit composé stimule ou inhibe l'activité enzymatique de la protéine de tyrosine phosphatase α du type récepteur.
